# EUROPEAN PATENT APPLICATION

(11) **EP 1 695 967 A1**
(43) Date of publication of application: **30.08.2006**
(21) Application number: 04799559.2
(22) Date of filing: 02.11.2004
(51) Int. Cl.: C07D 239/22, C07D 403/12, C07D 413/12, C07D 417/12, C12N 9/99

(54) **PROCESSES FOR PRODUCTION OF PYRIMIDINE DERIVATIVES AND INTERMEDIATES**

(30) Priority: 04.11.2003 JP 2003374043
(71) Applicant: Ajinomoto Co., Inc., Tokyo 104-8315 (JP)
(72) Inventor: TAKAHASHI, Daisuke, Ajinomoto Co., Inc., Kawasaki-shi, Kanagawa 2108681 (JP); IZAWA, Kunisuke, Ajinomoto Co., Inc., Kawasaki-shi, Kanagawa 2108681 (JP)
(74) Representative: Nicholls, Kathryn Margaret
(86) International application number: PCT/JP2004/016621
(87) International publication number: WO 2005/042499

(57) **Abstract**

Compound (I) is reacted with compound (II) to give compound (III), which is then reacted with compound (IX) to give compound (VIII), which is then preferably deprotected by an enzyme reaction to give compound (XII). The present invention provides an advantageous process for preparing a pyrimidine derivative useful as an enzyme inhibitor, and a synthetic intermediate: 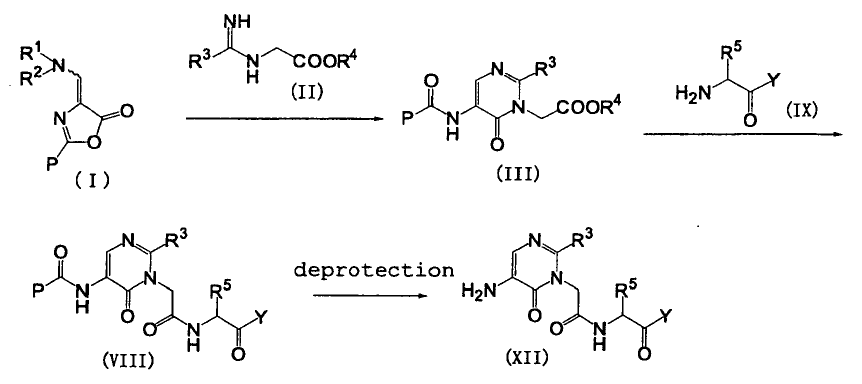 wherein P is an alkyl group and the like, R¹ and R² are alkyl groups and the like, R³ is an alkyl group optionally having substituent(s) and the like, R⁴ is a hydrogen atom and the like, R⁵ is an aralkyl group optionally having substituent(s) and the like, and Y is a heteroaryl group optionally having substituent(s) and the like.

## Description

### Technical Field

The present invention relates to a novel process for the preparation of a pyrimidine derivative useful as an enzyme inhibitor such as an elastase inhibitor, a chymase inhibitor and the like, and a synthetic intermediate therefor.

### Background Art

A group of compounds represented by the formula (XIII): 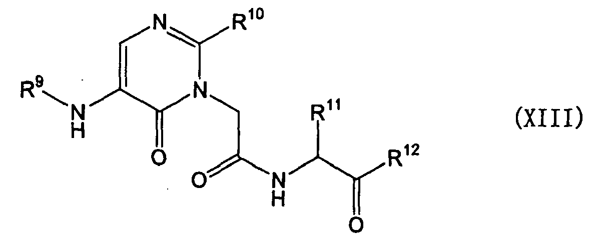 wherein R⁹ is a hydrogen atom, an acyl group, a formyl group and the like, R¹⁰ is a phenyl group optionally having substituent(s), an alkyl group and the like, R¹¹ is an alkyl group optionally having substituent(s), an aryl group optionally having substituent(s), an aralkyl group optionally having substituent(s) and the like, and R¹² is a heterocyclic group optionally having substituent(s), an acyl group, a haloalkyl group and the like have been reported to be useful as pharmaceutical products, particularly enzyme inhibitors such as an elastase inhibitor, a chymase inhibitor and the like for the prophylaxis or treatment of rheumatism, a chronic obstructive pulmonary disease and the like (WO98/24806 and WO98/09949).

As a process for preparing a compound represented by the formula (6), which is one of the compounds represented by the formula (XIII), a method depicted in the following reaction scheme has been reported (WO02/051815). 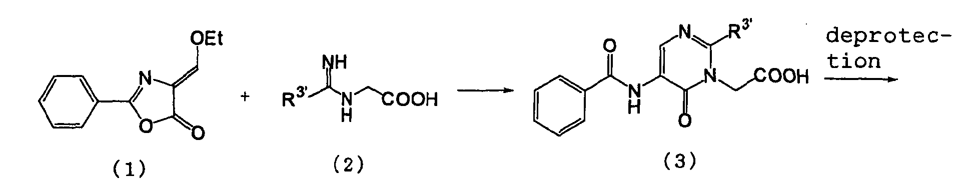 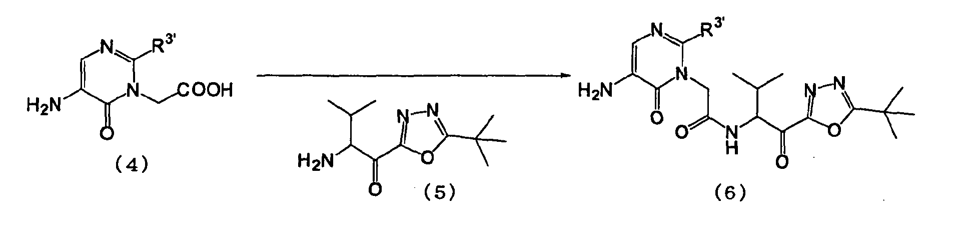 wherein R^{3'} is (1) C1-4 alkyl group, (2) Cycl or (3) C1-4 alkyl group substituted by Cycl (wherein Cycl is a C3-10 monocyclic or bicyclic carbon ring, a 3- to 10-membered monocyclic or bicyclic heterocycle containing 1 to 4 nitrogen atoms, 1 or 2 oxygen atoms and/or 1 or 2 sulfur atoms, etc.).

This method is associated with a problem of decreased yield and degraded quality of the object compound represented by the formula (6), because, when a compound represented by the formula (4) and a compound represented by the formula (5) are subjected to an amidation reaction, a side reaction of an amidation reaction with itself to generate a dimer of the compound represented by the formula (4) occurs due to the presence of an amino group at the 5-position of a pyrimidine ring of the compound represented by the formula (4).

In the above-mentioned method, the preparation of a dimer can be avoided when the amidation reaction of a compound represented by the formula (3) with a compound represented by the formula (5) is carried out without deprotection of a benzoyl group of a compound represented by the formula (3) and the benzoyl group is thereafter deprotected. However, deprotection of the benzoyl group after the amidation reaction requires severe conditions involving a strong base and refluxing for a long time (WO02/051815, Example 4 etc.). A pyrimidine compound represented by the formula (6) cannot be obtained easily since 1,3,4-oxadiazole ring in a compound represented by the formula (6) is decomposed under such conditions. To avoid dimerization reaction in the above-mentioned method, therefore, protection of an amino group of a compound represented by the formula (4) in advance with a protecting group capable of being deprotected under mild conditions may be performed. However, addition of further steps of protection and deprotection that have become necessary is not preferable.

In protection of amino group by acylation, it is generally known that hydrolysis of aliphatic acyl such as acetyl can proceed better under mild conditions than aromatic acyl such as benzoyl (Theodora W. Grrene, Peter G. M. Wuts, Protective Groups in Organic Synthesis, 2nd edn., A Wiley Interscience Publication, 1991, p. 349). Therefore, once a compound represented by the formula (3), wherein the 5-position amino group is protected by an aliphatic acyl group, is obtained, the compound may be amidated with a compound represented by the formula (5) without eliminating the aliphatic acyl group, and then deprotected under milder conditions free of decomposition.

For this end, a compound represented by the formula (1), wherein the 2-position of an azlactone ring is an aliphatic group, is necessary as a starting material. However, an efficient process for preparing such compound has not been reported yet.

The present inventors have tried synthesis of a compound represented by the formula (8), wherein the 2-position of an azlactone ring is a methyl group, using the Erlenmeyer method shown in the following reaction scheme known as a general process for preparing azlactone compounds.

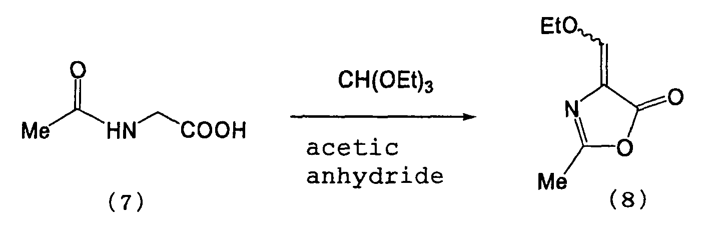

However, this method allows generation of a lot of byproducts, and an azlactone compound represented by the formula (8) was obtained only in an extremely low yield.

### Disclosure of the Invention

The problem to be solved by the present invention is provision of an advantageous process for preparing a pyrimidine derivative represented by the following formula (XII) or an N-protected form thereof (e.g., N-formyl form, see WO98/09949), which are useful as enzyme inhibitors and an intermediate thereof.

The present inventors have conducted intensive studies in an attempt to solve the above-mentioned problems and found that a compound represented by the formula (III) to be mentioned later can be surprisingly obtained even when a compound represented by the formula (I) to be mentioned later is used instead of a compound represented by the formula (1).

That is, they have found that, since a compound represented by the formula (I), wherein the 2-position of azlactone ring is an aliphatic group, is a known compound, and can be produced by a known method at a low cost and in a high yield (see Indian Journal of Chemistry, 39B, 200, 688-963), a compound represented by the formula (III), wherein the 5-position amino group of pyrimidine ring is protected by an aliphatic acyl, can be produced efficiently at a low cost by this method.

Moreover, they have studied a method of deprotection of a compound represented by the formula (VIII) to be mentioned later, which can be produced from this compound, and found a method capable of deprotection under mild conditions free of a side reaction such as decomposition and the like, which resulted in the completion of the present invention.

Accordingly, the present invention provides the following.
(1) A process for preparing a compound represented by the formula (III): 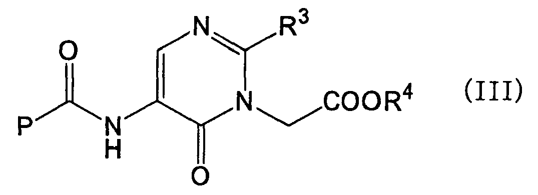 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), and R⁴ is a hydrogen atom, an alkyl group or an aralkyl group (hereinafter to be also referred to as compound (III)) or a salt thereof, which comprises reacting a compound represented by the formula (I): 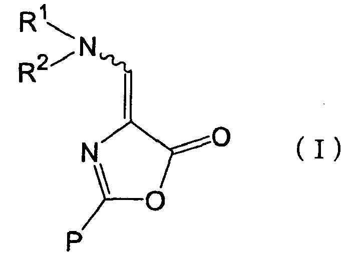 wherein R¹ and R² are the same or different and each independently is an alkyl group, or optionally form an aliphatic heterocycle together with the adjacent nitrogen atom, a wavy line shows a cis form or a trans form or a mixture thereof, and P is as defined above (hereinafter to be also referred to as compound (I)) with a compound represented by the formula (II): 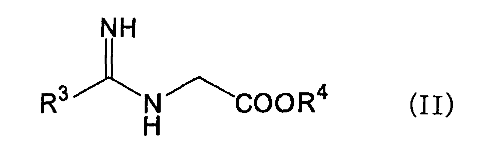 wherein each symbol is as defined above (hereinafter to be also referred to as compound (II)) or a salt thereof.
(2) A process for preparing a compound represented by the formula (IV): 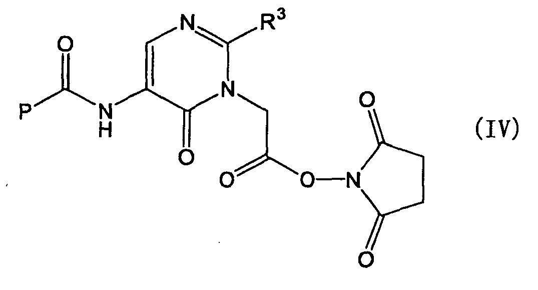 wherein each symbol is as defined above (hereinafter to be also referred to as compound (IV)), which comprises reacting compound (I) with compound (II) or a salt thereof to give compound (III) or a salt thereof; and
   condensing the obtained compound (III) or a salt thereof with N-hydroxysuccinimide after converting R⁴ to a hydrogen atom when it is other than a hydrogen atom.
(3) The process of the above-mentioned (1) or (2), wherein P is a methyl group, an ethyl group or a benzyl group.
(4) The process of the above-mentioned (1) or (2), wherein R³ is a phenyl group optionally having substituent(s) or a methyl group.
(5) The process of the above-mentioned (1) or (2), wherein R⁴ is a hydrogen atom, a methyl group or a tert-butyl group.
(6) A process for preparing a compound represented by the formula (VI): 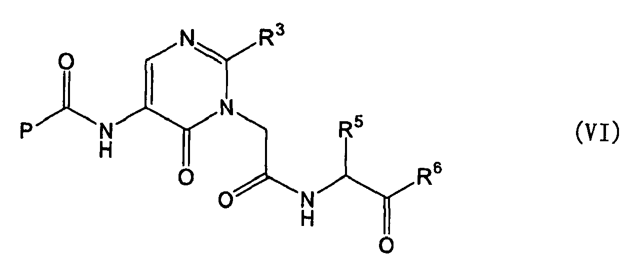 wherein R⁵ is a hydrogen atom, an alkyl group optionally having substituent(s), an aralkyl group optionally having substituent(s) or an aryl group optionally having substituent(s), R⁶ is a hydrogen atom, a hydroxyl group, an alkoxy group, an aralkyloxy group or -N(R⁷)-OR⁸ (wherein R⁷ and R⁸ are the same or different and each independently is an alkyl group), and other symbols are as defined above (hereinafter to be also referred to as compound (VI)) or a salt thereof, which comprises the following Steps (a), (b) and (c):
   Step (a): reacting compound (I) with compound (II) or a salt thereof to give compound (III) or a salt thereof;
   Step (b): condensing the obtained compound (III) or a salt thereof with N-hydroxysuccinimide after converting R⁴ to a hydrogen atom when it is other than a hydrogen atom, to give compound (IV); and
   Step (c): reacting the obtained compound (IV) with a compound represented by the formula (V): 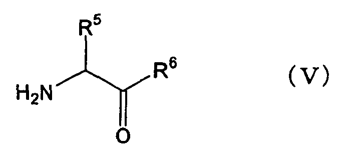
   wherein each symbol is as defined above (hereinafter to be also referred to as compound (V)) or a salt thereof to give compound (VI) or a salt thereof.
(7) A process for preparing compound (VI) or a salt thereof, which comprises reacting compound (I) with compound (II) or a salt thereof to give compound (III) or a salt thereof, and condensing the obtained compound (III) or a salt thereof with compound (V) or a salt thereof after converting R⁴ to a hydrogen atom when it is other than a hydrogen atom.
(8) The process of the above-mentioned (6) or (7), wherein P is a methyl group, an ethyl group or a benzyl group.
(9) The process of the above-mentioned (6) or (7), wherein R³ is a phenyl group optionally having substituent(s) or a methyl group.
(10) The process of the above-mentioned (6) or (7), wherein R⁴ is a hydrogen atom, a methyl group or a tert-butyl group.
(11) A process for preparing a compound represented by the formula (VII): 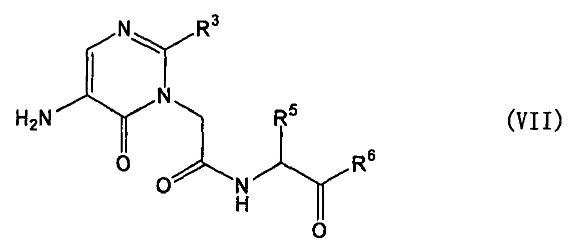 wherein each symbol is as defined above (hereinafter to be also referred to as compound (VII)) or a salt thereof, which comprises deprotecting compound (VI) or a salt thereof, which is obtained by the process of any one of the above-mentioned (6) to (10).
(12) The process of the above-mentioned (11), wherein the deprotection is carried out by an enzyme reaction.
(13) The process of the above-mentioned (11), wherein the deprotection is carried out under acidic conditions.
(14) A process for preparing a compound represented by the formula (XII): 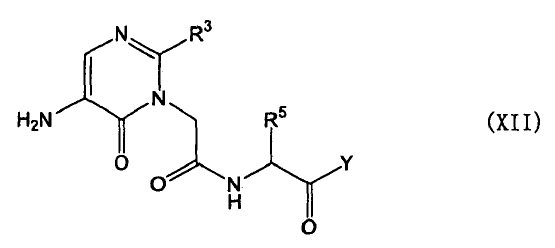 wherein Y is a heterocyclic group optionally having substituent(s), an acyl group optionally having substituent(s) or a haloalkyl group, and other symbols are as defined above (hereinafter to be also referred to as compound (XII)) or a salt thereof, which comprises the following Steps (h), (i) and (j) :
   Step (h): protecting an amino group of compound (VII) or a salt thereof, which is obtained by the process of any one of the above-mentioned (11) to (13) to give a compound represented by the formula (X): 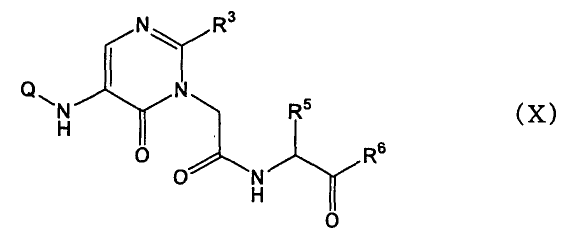 wherein Q is an amino-protecting group other than an acyl group, and other symbols are as defined above (hereinafter to be also referred to as compound (X)) or a salt thereof;
   Step (i): converting a group represented by R⁶ of the obtained compound (X) or a salt thereof to a group represented by Y to give a compound represented by the formula (XI): 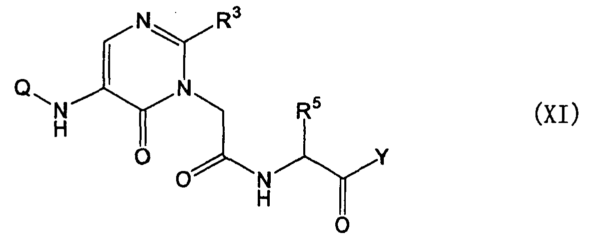 wherein each symbol is as defined above (hereinafter to be also referred to as compound (XI)) or a salt thereof; and
   Step (j): deprotecting the obtained compound (XI) or a salt thereof to give compound (XII) or a salt thereof.
(15) A process for preparing a compound represented by the formula (VIII) : 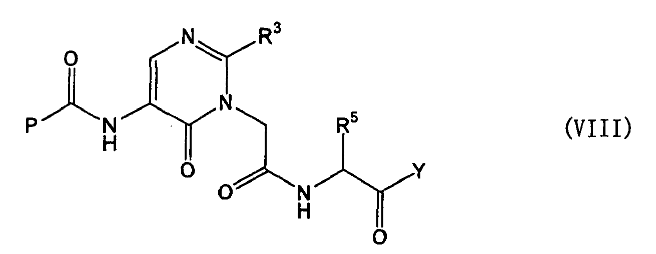 wherein each symbol is as defined above (hereinafter to be also referred to as compound (VIII)) or a salt thereof, which comprises the following Steps (a), (b), (c) and (e):
   Step (a): reacting compound (I) with compound (II) or a salt thereof to give compound (III) or a salt thereof;
   Step (b): condensing the obtained compound (III) or a salt thereof with N-hydroxysuccinimide after converting R⁴ to a hydrogen atom when it is other than a hydrogen atom to give compound (IV);
   Step (c): reacting the obtained compound (IV) with compound (V) or a salt thereof to give compound (VI) or a salt thereof; and
   Step (e): converting a group represented by R⁶ of the obtained compound (VI) or a salt thereof to a group represented by Y to give compound (VIII) or a salt thereof.
(16) A process for preparing compound (VIII) or a salt thereof, which comprises the following Steps (a), (d) and (e):
   Step (a): reacting compound (I) with compound (II) or a salt thereof to give compound (III) or a salt thereof;
   Step (d): condensing the obtained compound (III) or a salt thereof with compound (V) or a salt thereof after converting R⁴ to a hydrogen atom when it is other than a hydrogen atom, to give compound (VI) or a salt thereof; and
   Step (e): converting a group represented by R⁶ of the obtained compound (VI) or a salt thereof to a group represented by Y to give compound (VIII) or a salt thereof.
(17) A process for preparing compound (VIII) or a salt thereof, which comprises reacting compound (I) with compound (II) or a salt thereof to give compound (III) or a salt thereof, and condensing the obtained compound (III) or a salt thereof with a compound represented by the formula (IX): 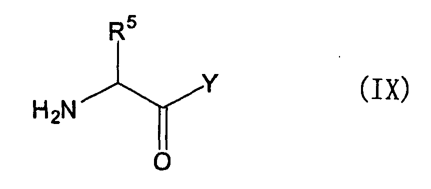 wherein each symbol is as defined above (hereinafter to be also referred to as compound (IX)) or a salt thereof, after converting R⁴ to a hydrogen atom when it is other than a hydrogen atom.
(18) The process of any one of the above-mentioned (15) to (17), wherein P is a methyl group, an ethyl group or a benzyl group.
(19) The process of any one of the above-mentioned (15) to (17), wherein R³ is a phenyl group optionally having substituent(s) or a methyl group.
(20) The process of any one of the above-mentioned (15) to (17), wherein R⁴ is a hydrogen atom, a methyl group or a tert-butyl group.
(21) A process for preparing compound (XII) or a salt thereof, which comprises deprotecting compound (VIII) or a salt thereof, which is obtained by the process of any one of the above-mentioned (15) to (20).
(22) The process of the above-mentioned (21), wherein the deprotection is carried out by an enzyme reaction.
(23) The process of the above-mentioned (21), wherein the deprotection is carried out under acidic conditions.

### Detailed Description of the Invention

The present invention is explained in detail in the following.

The definitions of the symbols used in the present invention are as follows.

The "alkyl group" for P, R¹, R², R⁴, R⁷ or R⁸ is a linear or branched chain alkyl group preferably having 1-20, more preferably 1-7, carbon atoms, such as methyl group, ethyl group, n-propyl group, isopropyl group, n-butyl group, isobutyl group, sec-butyl group, tert-butyl group, lauryl group and the like. Of these, methyl group and ethyl group are preferable.

The "alkyl group optionally having substituent(s)" for R³ or R⁵ is the above-mentioned alkyl group optionally substituted by one or more of the following substituents. As the substituent here, for example, a nitro group, a linear or branched chain alkoxy group (carbon atoms: 1-6, ex.: methoxy group), a halogen atom (ex.: chlorine atom, fluorine atom etc.), a hydroxyl group and the like can be mentioned.

The "alkenyl group" for P is a linear or branched chain alkenyl group preferably having 2-20, more preferably 2-7, carbon atoms, such as vinyl group, allyl group, homoallyl group, oleyl group and the like, with preference given to vinyl group and allyl group.

The "aryl group optionally having substituent(s)" for R⁵ is an aryl group preferably having 6-20, more preferably 6-8, carbon atoms. The aryl group is optionally substituted by one or more of the following substituents. As the substituent in this context, for example, a nitro group, a linear or branched chain alkoxy group (carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), a hydroxyl group and the like can be mentioned, and as the substituent of the "phenyl group optionally having substituent(s)" for R³, similar ones can be mentioned. Specific examples of an aryl group optionally having substituent(s) and a phenyl group optionally having substituent(s) include phenyl group, o-, m- or p-nitrophenyl group, o-, m- or p-methoxyphenyl group, o-, m- or p-chlorophenyl group, o-, m- or p-fluorophenyl group, o-, m- or p-tolyl group and the like, with preference given to phenyl group, p-fluorophenyl group and p-tolyl group.

The "aralkyl group" for R⁴ is an aralkyl group wherein the aryl moiety is an aryl group preferably having 6-12, more preferably 6-8, carbon atoms, and the alkyl moiety is a linear or branched chain alkyl group preferably having 1-6, more preferably 1-3, carbon atoms. Specific examples of aralkyl group preferably include benzyl group.

The "aralkyl group optionally having substituent(s)" for P or R⁵ is the above-mentioned aralkyl group optionally substituted by one or more of the following substituents, and the substituent in this context includes, for example, a nitro group, a linear or branched chain alkoxy group (carbon number: 1-6, e.g.: methoxy group), a halogen atom (e.g.: chlorine atom, fluorine atom etc.), a hydroxyl group and the like.

The "haloalkyl group" for P and Y is a straight chain or branched chain alkyl group preferably having 1-5, more preferably 1-3, carbon atoms, which is substituted by one or more halogen atoms (fluorine atom, chlorine atom, bromine atom, iodine atom). For example, trifluoromethyl group, trichloromethyl group, chloromethyl group and the like can be mentioned, with preference given to trifluoromethyl group.

As the "aliphatic heterocycle" optionally formed by R¹ and R² together with the adjacent nitrogen atom is a 5- or 6-membered aliphatic heterocycle containing a carbon atom and at least one nitrogen atom, and optionally containing 1 to 3 hetero atoms selected from oxygen atom, sulfur atom and nitrogen atom. For example, pyrrolidine, piperidine, morpholine, thiomorpholine, piperazine and the like can be mentioned.

The "alkoxy group" for R⁶ is a linear or branched chain alkoxy preferably having 1-20, more preferably 1-7, carbon atoms, such as methoxy group, ethoxy group, n-propoxy group, isopropoxy group, n-butoxy group, isobutoxy group, sec-butoxy group, tert-butoxy group, lauryloxy group and the like. Of these, methoxy group and ethoxy group are preferable.

The "aralkyloxy group" for R⁶ is an aralkyloxy group having the aralkyl moiety defined above, such as benzyloxy group, p-nitrobenzyloxy group and the like.

The "hetero ring group" of the "heterocyclic group optionally having substituent(s)" for Y is a 5- or 6-membered heterocyclic group, containing, besides carbon atom, 1-5 hetero atoms selected from oxygen atom, sulfur atom and nitrogen atom, or a condensed hetero ring group thereof and the like. For example, pyrrolyl group (1-, 2- or 3-pyrrolyl group), furyl group (2- or 3-furyl group), thienyl group (2- or 3-thienyl group), imidazolyl group (1-, 2-, 4- or 5-imidazolyl group), oxazolyl group (2-, 4- or 5-oxazolyl group), thiazolyl group (2-, 4- or 5-thiazolyl group), pyrazolyl group (1-, 3-, 4- or 5-pyrazolyl group), isoxazolyl group (3-, 4- or 5-isoxazolyl group), isothiazolyl group (3-, 4- or 5-isothiazolyl group), oxadiazolyl group (1,2,4-oxadiazol-3 or 5-yl group, 1,3,4-oxadiazol-2-yl group, 1,2,5-oxadiazol-3-yl group), thiadiazolyl group, (1,2,4-thiadiazol-3 or 5-yl group, 1,3,4-thiadiazol-2-yl group, 1,2,5-thiadiazol-3-yl group), triazolyl group (1,2,4-triazol-1, 3, 4 or 5-yl group, 1,2,3-triazol-1, 2 or 4-yl group), indolyl group (1-, 2-, 3-, 4-, 5-, 6- or 7-indolyl group), benzofuryl group (2-, 3-, 4-, 5-, 6- or 7-benzofuryl group), benzothienyl group (2-, 3-, 4-, 5-, 6- or 7-benzothienyl group), benzimidazolyl group (1-, 2-, 4-, 5-, 6-or 7-benzimidazolyl group), benzoxazolyl group (2-, 4-, 5-, 6-or 7-benzoxazolyl group), benzothiazolyl group (2-, 4-, 5-, 6-or 7-benzothiazolyl group), pyridyl group (2-, 3- or 4-pyridyl group), 1-oxidopyridyl group (1-oxido-2-, 3- or 4-pyridyl group), pyrimidinyl group (2-, 4- or 5-pyrimidinyl group), tetrazolyl group (1H-tetrazol-1 or 5-yl group, 2H-tetrazol-2 or 5-yl group), quinolyl group (2-, 3-, 4-, 5-, 6-, 7- or 8-quinolyl group) or isoquinolyl group (1-, 3-, 4-, 5-, 6-, 7- or 8-isoquinolyl group), pyrrolidinyl group (1-, 2- or 3-pyrrolidinyl group), pyrazolidinyl group (1-, 3- or 4-pyrazolidinyl group), imidazolidinyl group (1-, 2- or 4-imidazolidinyl group), pyrrolinyl group (1-, 2- or 3-pyrrolinyl group), pyrazolinyl group (1-, 3- or 4-pyrazolinyl group), imidazolinyl group (1-, 2-, 4- or 5-imidazolinyl group), tetrahydrofuryl group (2- or 3-tetrahydrofuryl group), tetrahydrothienyl group (2- or 3-tetrahydrothienyl group), thiazolidinyl group (2-, 3-, 4- or 5-thiazolidinyl group), piperidinyl group (1-, 2-, 3- or 4-piperidinyl group), piperazinyl group (1- or 2-piperazinyl group), tetrahydropyranyl group (2-, 3- or 4-tetrahydropyranyl group), morpholinyl group (2-, 3- or 4-morpholinyl group), thiomorpholinyl group (2-, 3- or 4-thiomorpholinyl group), dioxolanyl group (2- or 4-dioxolanyl group), homopiperidinyl group (1-, 2-, 3- or 4-homopiperidinyl group), homopiperazinyl group (1-, 2-, 5- or 6-homopiperazinyl group), indolinyl group (1-, 2-, 3-, 4-, 5-, 6- or 7-indolinyl group), chromanyl group (2-, 3-, 4-, 5-, 6-, 7- or 8-chromanyl group) and the like can be mentioned, oxadiazolyl group, thiazolyl group and benzoxazolyl group are preferable and 1,3,4-oxadiazol-2-yl group, 2-thiazolyl group and 2-benzoxazolyl group are more preferable.

The hetero ring group is optionally substituted by one or more of the following substituents. As the substituent here, for example, a nitro group, a linear or branched chain alkoxy group (preferable carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), alkoxycarbonyl group (e.g.: methoxycarbonyl group, ethoxycarbonyl group etc.) and the like can be mentioned.

Specific examples of the heterocyclic group optionally having substituent(s) include 5-tert-butyl-1,3,4-oxadiazol-2-yl group, 2-thiazolyl group, 5-methoxycarbonylbenzoxazol-2-yl group and the like, with preference given to 5-tert-butyl-1,3,4-oxadiazol-2-yl group.

The "acyl group" of the "acyl group optionally having substituent(s)" for Y is a linear or branched chain acyl group preferably having 1-20 carbon atoms, such as acetyl group, propionyl group, butyryl group, isobutyryl group and the like. The acyl group is optionally substituted by one or more of the following substituents. As the substituent here, for example, a nitro group, a linear or branched chain alkoxy group (preferable carbon number: 1-6, e.g.: methoxy group), a halogen atom (e.g.: chlorine atom, fluorine atom etc.), a linear or branched chain alkyl group (preferable carbon number: 1-4, e.g.: methyl group, ethyl group, propyl group etc.), a 2-pyridyloxy group and the like can be mentioned. Specific examples of the "acyl group optionally having substituent(s)" preferably include 4-(2-pyridyloxy)butyryl group.

The "amino-protecting group other than acyl group" for Q is not particularly limited as long as it is other than acyl group. For example, benzyloxycarbonyl group (Cbz group), tert-butoxycarbonyl group (Boc group), methoxycarbonyl group (Moc group), 9-fluorenylmethoxycarbonyl group (Fmoc group) and the like can be mentioned. Of these, Cbz group, Boc group and the like permitting deprotection under mild conditions are preferable.

Preferable embodiment of each symbol is explained in the following.

P is preferably an alkyl group or an aralkyl group optionally having substituent(s), and methyl group, ethyl group and benzyl group are more preferable.

As R³, a phenyl group, a p-fluorophenyl group or a methyl group is preferable.

R⁴ is preferably a hydrogen atom or an alkyl group, and a hydrogen atom, a methyl group or a tert-butyl group is more preferable.

R⁵ is preferably an alkyl group optionally having substituent(s) or an aralkyl group optionally having substituent(s), and methyl group, isopropyl group or benzyl group is more preferable.

R⁶ is preferably a hydrogen atom, a hydroxyl group, methoxy group, tert-butoxy group, benzyloxy group or -N(R⁷)-OR⁸ (each symbol is as defined above), and hydrogen atom, methoxy group, benzyloxy group or -N(Me)-OMe is more preferable.

Y is preferably 5-tert-butyl-1,3,4-oxadiazol-2-yl group, 2-thiazolyl group, trifluoromethyl group, 4-(2-pyridyloxy)butyryl group or 5-methoxycarbonylbenzoxazol-2-yl group.

When compounds represented by the formulas (II), (III), (V), (VI), (VII), (VIII), (IX), (X), (XI) and (XII) have an acidic group, salts such as alkali metal salt (e.g., potassium salt, sodium salt, lithium salt etc.), organic amine salt (e.g., triethylamine salt, dicyclohexylamine salt etc.) and the like can be formed, and when the compounds have a basic group; inorganic acid salt (e.g., hydrochloride, sulfate etc.), organic acid salt (e.g., acetate, trifluoroacetate, tosylate, mesylate etc.) and the like can be formed.

The process for preparing the present invention is shown in the following reaction schemes. 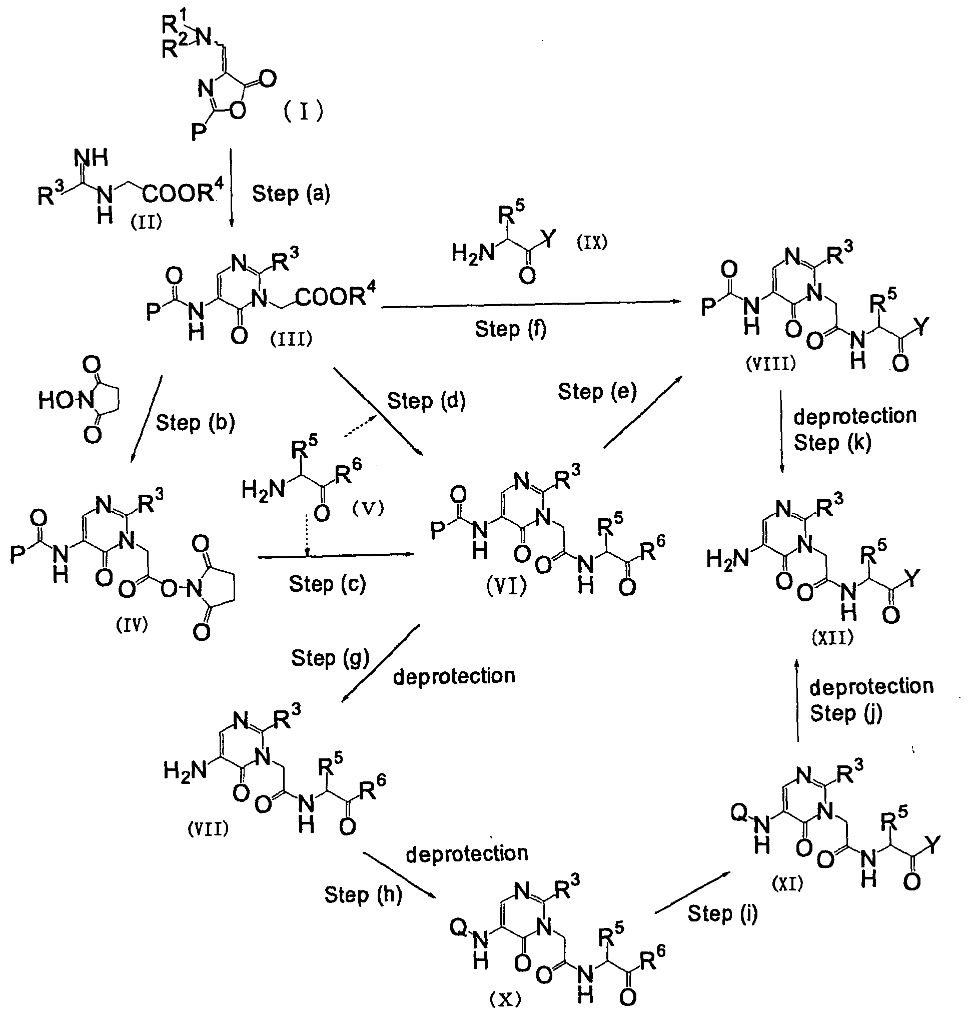 wherein each symbol is as defined above.

In other words, the present invention relates to a process for preparing a compound represented by the formula (III), (IV), (VI), (VII), (VIII) or (XII), which partially includes Steps (a)-(k) shown in the above-mentioned reaction scheme, and a preferable route for preparing a desired compound can be appropriately selected.

The process for preparing the present invention comprises a novel synthetic route, Step (a) for producing compound (III), wherein the amino group at the 5-position on the pyrimidine ring is protected by an aliphatic acyl, and by this step, compound (III) can be produced efficiently.

Using compound (III) as a starting material of pyrimidine compound (XII) or (VII), which is an enzyme inhibitor or a useful intermediate therefore, the reaction can proceed via compound (IV), (VI) and (VIII), which are synthetic intermediates wherein the 5-position amino group of pyrimidine ring is protected by aliphatic acyl permitting deprotection under mild conditions, deprotection can be performed without decomposition and the like in Step (g) or (k).

As a result, deprotection prior to Steps (b), (c), (d), (e) and (f) is not necessary, and side reactions such as dimerization by condensation reaction in the form of a free amino group and the like can be avoided. In addition, since the protecting group does not need to be changed to avoid dimerization, compound (VII) or compound (XII) having high quality can be efficiently prepared in a smaller number of steps.

In the present invention, moreover, it has been found that deprotection can be performed by an enzyme reaction in final deprotection Step (g) or (k) by using compound (III) as a starting material. Since the enzyme reaction proceeds in a high yield under mild conditions by stirring at room temperature in around a neutral range, it can be preferably applied to the present invention.

It has been further found that deprotection proceeds in a high yield under mild conditions of weak acidic near room temperature without accompanying side reactions such as decomposition and the like.

Steps (a)-(k) are explained in the following.

### 1. Step (a)

In Step (a), compound (I) is reacted with compound (II) or a salt thereof to give compound (III). This reaction is carried out in a solvent. To be specific, for example, compound (I) or a solution thereof is added to compound (II) or a solution thereof (when compound (I) is a solution, dropwise addition is preferable), and the mixture is stirred with heating. The order of addition may be reverse or simultaneous.

Compound (II) wherein R⁴ is an alkyl group or an aralkyl group tends to be decomposed in a free form and, as a solid, it is generally isolated in the form of a stable salt. As the salt of compound (II), for example, acid addition salts such as hydrochloride, sulfate, trifluoroacetate and the like can be mentioned. Compound (II) wherein R⁴ is a hydrogen atom is stably present as a salt formed with the imino group in a molecule, and is also stably present as a base addition salt such as sodium salt, potassium salt and the like. When compound (II) in a free form is to be reacted with compound (I), a base (e.g., alkoxide compounds such as sodium ethoxide, potassium butoxide, sodium methoxide and the like) is preferably used in an amount sufficient to convert compound (II) to a base addition salt.

In Step (a), it is preferable to neutralize the acid addition salt of compound (II) with a base in a solvent to once convert same to a free form, which is then reacted with compound (I). While it is possible, for example, to dissolve a salt of compound (II) and compound (I) in a solvent, without once forming a free form before reaction, and allow reaction by adding a base, the yield of the object compound (III) decreases in this case. The base to be used for neutralization is not particularly limited and, for example, sodium hydroxide, potassium hydroxide, sodium carbonate, potassium carbonate, triethylamine, sodium methoxide, sodium ethoxide and the like can be mentioned. These bases can also be used in the form of an aqueous solution. The neutralization is generally carried out in a solvent. As the solvent, toluene, ethyl acetate and the like can be used. In this case, water is preferably added to solve the base. The free form of compound (II), which is extracted in an organic layer, can be partitioned and used. While the amount of the base to be used is not particularly limited as long as a salt of compound (II) can be converted to a free form thereof, it is preferably not more than 3 equivalents per compound (II) from an economical aspect.

As the solvent to be used in Step (a), any solvent can be used as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, tetrahydrofuran (THF), alcohols (e.g., ethanol, isopropyl alcohol, n-butanol and the like), N,N-dimethylformamide and the like can be mentioned. These may be used in combination of two or more kinds thereof, and N,N-dimethylformamide is preferable. The amount of solvent to be used is generally 2- to 50-fold weight, preferably 5- to 20-fold weight, relative to compound (I).

The amount of compound (II) to be used is generally 0.9-3 equivalents, preferably 1-1.3 equivalents, per compound (I).

The reaction of compound (I) and compound (II) is generally carried out within the temperature range of from 0°C to the refluxing temperature of the solvent to be used (preferably 60-130°C). The reaction is generally completed for 2 hr-30 hr (preferably 5 hr-20 hr) within the above-mentioned temperature range.

Compound (III) can be isolated and purified by a conventional method. For example, after the completion of the reaction, the reaction mixture is washed successively with an aqueous acidic solution (e.g., hydrogen chloride, sulfuric acid etc.) and an aqueous alkali solution (e.g., saturated aqueous sodium hydrogen carbonate, brine etc.) and partitioned, and the obtained organic layer is concentrated to isolate compound (III). Furthermore, compound (III) can be purified by adding a crystal precipitation solvent such as ethers (e.g., diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc. to allow crystal precipitation or subjecting the compound to silica gel column chromatography, but the method is not limited to these.

### 2. Step (b)

In Step (b), when R⁴ is other than a hydrogen atom, compound (III) or a salt thereof is converted to a carboxylic acid form by hydrolysis and the like and condensed with N-hydroxysuccinimide to give compound (IV).

Since compound (IV) can be amidated as it is with various amines as shown in the following Step (c), it is convenient as a versatile intermediate and can be advantageously condensed with an amine having a free carboxyl group such as amino acid, without protecting the carboxyl group.

Compound (III) wherein R⁴ is other than a hydrogen atom, i.e., an alkyl group or an aralkyl group, is first converted to the form of carboxylic acid (R⁴= hydrogen atom, hereinafter to be simply referred to as carboxylic acid form) by subjecting the compound to hydrolysis, acid treatment, hydrogenation and the like. The hydrolysis, acid treatment, hydrogenation and the like may be carried out by a method known to those of ordinary skill in the art. For example, hydrolysis can be carried out by reacting the compound with a base (e.g., sodium hydroxide, potassium hydroxide etc.) or an acid (e.g., hydrochloric acid, sulfuric acid etc.) in an alcohol (e.g., methanol and ethanol etc.) or a mixed solvent with water, but the method is not limited to these.

For example, when R⁴ is a tert-butyl group and the like, compound (III) can be converted to a carboxylic acid form by an acid treatment, for example, by treating with an acid such as trifluoroacetic acid and the like in an organic solvent.

When R⁴ is benzyl and the like, compound (III) can be converted to a carboxylic acid form by hydrogenolysis, for example, by hydrogenation in the presence of a catalyst such as palladium carbon and the like.

The method of condensing the carboxylic acid form and N-hydroxysuccinimide is not particularly limited and can be a method known to those of ordinary skill in the art. For example, the carboxylic acid form may be reacted with a chlorinating agent such as thionyl chloride, phosphorus pentachloride and the like to give an acid chloride, which may be condensed with N-hydroxysuccinimide. However, since primary amide is present in the molecule of compound (III), a method including activating the carboxylic acid form with a condensing agent and condensing the compound with N-hydroxysuccinimide is preferable.

While a preferable embodiment is explained in the following, Step (b) is not limited to this embodiment.

In a preferable concrete embodiment, the carboxylic acid form is activated with an activation agent in a solvent and then condensed with N-hydroxysuccinimide.

Examples of the activation agent include chloroformates (e.g., ethyl chloroformate, isobutyl chloroformate, methyl chloroformate etc.), carbodiimides (e.g., N,N'-dicyclohexylcarbodiimide (DCC), N-(3-dimethylaminopropyl)-N'-ethylcarbodiimide (EDC) etc.), carbodiimidazole (CDI), diphenylphosphoryl azide (DPPA) and the like, but the activation agent is not limited to those. Of these, chloroformates, DCC, EDC and the like are preferable, and ethyl chloroformate and isobutyl chloroformate are particularly preferable.

The amount of the activation agent to be used is generally 0.9-1.5 equivalents, preferably 1-1.3 equivalents, relative to carboxylic acid form.

When the carboxylic acid compound is activated, a base may be added as necessary. As such base, inorganic base (e.g., sodium carbonate, potassium carbonate etc.) and organic base (e.g., triethylamine, diisopropylethylamine, N-methylmorpholine, pyridine, piperidine etc.) can be mentioned, with preference given to organic base, particularly N-methylmorpholine, triethylamine and pyridine.

The amount of the base to be used is generally 0.9-2 equivalents, preferably 1-1.5 equivalents, relative to the carboxylic acid form.

The solvent to be used for the activation of the carboxylic acid form may be any as long as it does not inhibit the reaction and, for example, acetates (e.g., ethyl acetate, isopropyl acetate, isobutyl acetate, n-butyl acetate and the like), hydrocarbons (e.g., toluene, benzene, xylene and the like), acetonitrile, THF, dichloromethane, methyl-tert-butyl ether and the like can be mentioned, which may be used alone or in a combination of two or more kinds thereof. A single or mixed solvent of acetonitrile, THF, ethyl acetate and the like is more preferable. The amount of the solvent to be used is generally 3-50 fold weight, preferably 5-20 fold weight, relative to the carboxylic acid form.

The carboxylic acid form is activated within the temperature range of generally from -40°C to the refluxing temperature of the solvent to be used (preferably -10 to 10°C). This activation completes within the above-mentioned temperature range generally for 5 min-3 hr (preferably 10 min-1 hr).

After the activation of the carboxylic acid form, N-hydroxysuccinimide is added to the reaction mixture. The order of addition may be reverse or simultaneous.

The amount of N-hydroxysuccinimide to be used is generally 0.9-2 equivalents, preferably 1-1.3 equivalents, relative to the carboxylic acid form.

The condensation with N-hydroxysuccinimide is carried out within the temperature range of generally -50 to 40°C (preferably -20 to 20°C). The condensation completes within the above-mentioned temperature range generally for 1 hr-20 hr (preferably 2 hr-5 hr).

Isolation and purification of compound (IV) can be carried out by a conventional method. For example, after the completion of the reaction, the reaction mixture is washed successively with an aqueous acidic solution (e.g., hydrogen chloride, sulfuric acid etc.) or an aqueous alkali solution (e.g., saturated aqueous sodium hydrogen carbonate, brine etc.), partitioned and the obtained organic layer is concentrated to isolate compound (IV). Furthermore, compound (IV) can be purified by adding a crystal precipitation solvent such as ethers (e.g., diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc. to allow crystal precipitation or subjecting the compound to silica gel column chromatography, but the method is not limited to these.

### 3. Step (c)

In Step (c), compound (IV) is reacted with compound (V) or a salt thereof to give compound (VI) or a salt thereof. To be specific, for example, compound (IV) or a solution thereof is added to a solution of compound (V) in a solvent. The order of addition may be reverse or simultaneous.

Step (c) is advantageous in that the step can be carried out even for compound (V) wherein R⁶ is a hydroxyl group, without side reactions such as self-polymerization and the like of compound (V).

Therefore, as compound (V) in Step (c), a compound (V) wherein R⁶ is a hydroxyl group, or a free amino acid can be preferably used.

The solvent usable in Step (c) may be any as long as it does not inhibit the reaction and, for example, water, acetates (e.g., ethyl acetate, isopropyl acetate, isobutyl acetate, n-butyl acetate and the like), acetonitrile, THF, N,N-dimethylformamide, acetone and the like can be mentioned, which may be used alone or in a combination of two or more kinds thereof. A single or mixed solvent of water, acetonitrile, THF and the like is more preferable. The amount of the solvent to be used is generally 2 to 50-fold weight, preferably 5 to 20-fold weight, relative to the compound (IV).

When compound (V) is a free amino acid, it is preferably used as an aqueous solution at pH 5-10 obtained in advance with an alkaline aqueous solution (e.g., aqueous sodium hydroxide solution, aqueous potassium hydroxide solution etc.). The amount of water in the aqueous solution is contained in the amount of the solvent to be used.

The amount of compound (V) to be used is generally 0.8-2 equivalents, preferably 1-1.5 equivalents, relative to the compound (IV).

Step (c) is generally carried out within the temperature range of from -20°C to the refluxing temperature of the solvent to be used (preferably 0-30°C). This reaction completes within the above-mentioned temperature range generally for 30 min to overnight (preferably 1 hr-10 hr).

When R⁶ of compound (VI) is a hydroxyl group, the compound is present in the form of a carboxylic acid salt. Thus, a free form of compound (VI) can be purified by adding an acid (e.g., hydrochloric acid, sulfuric acid etc.) to the reaction mixture to adjust pH to not more than 3 and, for example, concentrating the reaction mixture or allowing crystal precipitation by adding a crystal precipitation solvent (e.g., ethers (e.g., diethyl ether, THF and the like), acetone, acetonitrile, hydrocarbon solvents (e.g., toluene, benzene, hexane, heptane and the like), halogen solvents (e.g., dichloromethane, dichloroethane and the like), alcohols (e.g., methanol, ethanol, isopropanol and the like), water, a mixed solvent thereof and the like), or subjecting the compound to silica gel column chromatography, but the method is not limited to these.

When R⁶ of compound (VI) is other than a hydroxyl group, the compound can be isolated or purified by a conventional method. For example, compound (VI) can be isolated by washing, after the completion of reaction, the reaction mixture successively with an acidic aqueous solution (e.g., hydrochloric acid, sulfuric acid and the like) and an aqueous alkaline solution (e.g., saturated aqueous sodium hydrogen carbonate, brine and the like), and concentrating the organic layer obtained by partitioning. Furthermore, compound (VI) can be purified by adding a crystal precipitation solvent (e.g., ethers (e.g., diethyl ether, THF and the like), acetone, acetonitrile, hydrocarbon solvents (e.g., toluene, benzene, hexane, heptane and the like), halogen solvents (e.g., dichloromethane, dichloroethane and the like), alcohols (e.g., methanol, ethanol, isopropanol and the like), water, a mixed solvent thereof and the like) to allow crystal precipitation, or subjecting the compound to silica gel column chromatography, but the method is not limited to these.

### 4. Step (d)

In Step (d), when R⁴ is other than a hydrogen atom, compound (III) or a salt thereof is converted to a carboxylic acid form, and condensed with compound (V) to give compound (VI) or a salt thereof.

Compound (VI) synthesized in Step (c) and Step (d) is useful as a synthetic intermediate capable of efficiently producing compound (XII), which is an enzyme inhibitor, because a group represented by R⁶ can be converted to a group represented by Y in the below-mentioned Step (e) while the amino group of compound (V) is protected.

In Step (d), compound (V) wherein R⁶ is a hydroxyl group is not preferable because side reactions such as self-polymerization and the like may occur.

Therefore, Step (d) can be preferably applied to compound (V) wherein R⁶ is other than a hydroxyl group, namely, a hydrogen atom, an alkoxy group, an aralkyloxy group or-N(R⁷)-OR⁸ wherein each symbol is as defined above.

When R⁴ of compound (III) is other than a hydrogen atom, namely, when it is an alkyl group or an aralkyl group, the compound is first converted to a carboxylic acid form by a method similar to that in Step (b).

For condensation of the carboxylic acid form and compound (V), the method of Step (b) for the condensation of the carboxylic acid form and N-hydroxysuccinimide is applied using compound (V) or a salt thereof instead of N-hydroxysuccinimide.

That is, in a preferable embodiment, a carboxylic acid form is activated with an activation agent in a solvent, and condensed with compound (V) or a salt thereof to give compound (VI).

While this embodiment is explained in the following, Step (d) is not limited to this embodiment.

As the activation agent, those similar to the activation agents used in Step (b) can be mentioned, and chloroformates, particularly ethyl chloroformate, methyl chloroformate, isobutyl chloroformate and the like are preferable.

The amount of the activation agent to be used is generally 0.9-1.5 equivalents, preferably 1-1.2 equivalents, relative to the carboxylic acid form.

During the activation of the carboxylic acid form, a base may be added as necessary. As such a base, the bases similar to those used in Step (b) can be mentioned, with preference given to N-methylmorpholine, triethylamine, pyridine and piperidine.

The amount of the base to be used is generally 0.9-2 equivalents, preferably 1-1.2 equivalents, relative to the carboxylic acid form.

The solvent to be used for the activation of a carboxylic acid form may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, halogen solvents (e.g., dichloromethane, chloroform etc.), ether solvents (e.g., diethyl ether, methyl-tert-butyl ether etc.) and the like can be mentioned. These may be used in combination of two or more kinds thereof, and a single or mixed solvent of acetonitrile, THF, ethyl acetate, methyl-tert-butyl ether and the like is more preferable. The amount of the solvent to be used is generally 3- to 50-fold weight, preferably 5- to 20-fold weight, relative to the carboxylic acid form.

A carboxylic acid form is activated within the range of generally from -50°C to -40°C (preferably -20°C to 20°C). The activation completes within the above-mentioned temperature range generally for 5 min-5 hr (preferably 15 min - 2 hr).

After the activation of the carboxylic acid form, compound (V) is added to the reaction mixture. The order of addition may be reverse or simultaneous.

The amount of compound (V) to be used is generally 0.9-3 equivalents, preferably 1-1.5 equivalents, relative to the carboxylic acid form.

When compound (V) is a salt, a base similar to the one used for activation of the carboxylic acid form is preferably added concurrently for neutralization.

The amount of the base to be used is generally 0.9-2 equivalents, preferably 1-1.5 equivalents, relative to the compound (V).

The condensation with compound (V) is performed within the temperature range of generally from -50°C to 40°C (preferably -20°C to 20°C). The condensation completes within the above-mentioned temperature range generally for 0.5 hr-20 hr (preferably 1 hr-3 hr).

Compound (VI) can be isolated and purified by a conventional method. After the completion of the reaction, for example, the reaction mixture is washed successively with an aqueous acidic solution (e.g., hydrogen chloride, sulfuric acid etc.), an aqueous alkali solution (e.g., saturated aqueous sodium hydrogen carbonate, brine etc.) and partitioned, and the obtained organic layer is concentrated to isolate compound (VI). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (VI), but the method is not limited to these.

As compound (V), which is a starting material for Step (c) and Step (d), commercially available amino acids can be used, or compound (V) can be produced by esterifying them by a known method, or by condensing them with a hydroxylamine derivative represented by the formula: NH(R⁷)-OR⁸, wherein each symbol is as defined above, by a known method.

### 5. Step (e)

In Step (e), a group represented by R⁶ of compound (VI) or a salt thereof is converted to a group represented by Y to give compound (VIII) or a salt thereof.

Step (e) can be one of the advantageous reaction routes for efficient production of compound (XII), because, as mentioned above, compound (VIII) can be produced by converting a group represented by R⁶ to a group represented by Y while the amino group of compound (VI) is protected.

Since Step (e) employs different methods for converting a group represented by R⁶ to a group represented by Y depending on the embodiment of Y, this step is explained below for each case. 5-1. When Y is heterocyclic group optionally having substituent(s) (hereinafter to be also referred to as Step (e-1))

In Step (e-1), as shown in the following reaction scheme, a group represented by R⁶ is converted to a heterocyclic group. To be specific, compound (VI) is reacted with a reagent represented by the formula (XIVa): Y¹-M¹ wherein Y¹ is a heterocyclic group optionally having substituent(s) and M¹ is lithium or MgX (wherein X is a chlorine atom, a bromine atom or an iodine atom) (hereinafter to be also referred to as reagent (XIVa)) in a solvent to give a compound represented by the formula (VIIIa) wherein Y is a heterocyclic group optionally having substituent(s) (hereinafter to be also referred to as compound (VIIIa)). 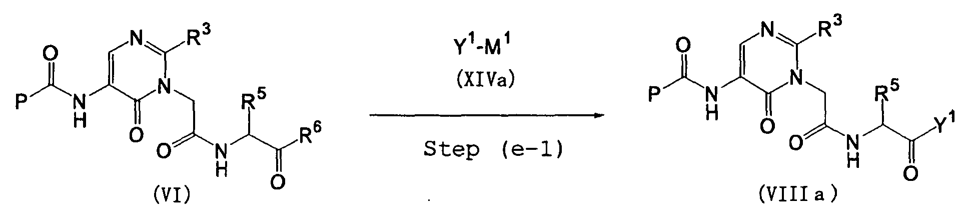 wherein each symbol is as defined above.

In Step (e-1), R⁶ of compound (VI) is preferably a group represented by -N(R⁷)-OR⁸ wherein each symbol is as defined above, to prevent further reaction of compound (VIIIa) with reagent (XIVa).

The reagent (XIVa) can be obtained by reacting a compound represented by the formula: Y¹-H or Y¹-X wherein each symbol is as defined above with alkyl lithiums (e.g., n-butyl lithium, methyl lithium etc.) or lithium amides (e.g., lithium diisopropylamide etc.) in ether solvents (e.g., THF, diethyl ether, methyl-tert-butylether (MTBE) etc.) at -90°C to 20°C, or reacting a compound represented by the formula: Y¹-X wherein each symbol is as defined above with magnesium under the general conditions for forming a Grignard reagent.

The solvent usable for Step (e-1) may be any as long as it does not inhibit the reaction and, for example, ether solvents (e.g., THF, diethyl ether, MTBE etc.), hydrocarbon solvents (e.g., n-hexane, toluene etc.) and the like can be mentioned, which may be used alone or in a combination of two or more kinds thereof. The amount of the solvent to be used is generally 5 to 50-fold weight, preferably 8 to 20-fold weight, relative to the compound (VI).

The amount of the reagent (XIVa) to be used is generally 1-10 equivalents, preferably 1-6 equivalents, relative to the compound (VI).

Step (e-1) is carried out within the temperature range of generally from -90°C to 30°C (preferably -80°C to -20°C). The reaction completes within the above-mentioned temperature range generally for 10 min-5 hr (preferably 30 min-2 hr).

When R⁶ is a hydrogen atom, reduced compound (VIIIa) (alcohol form) is obtained, which is then oxidized by a known method (e.g., Dess-Martin reagent, Swern oxidation, Pfitzner-Moffatt oxidation etc.) to give compound (VIIIa). 5-2. When R⁶ is hydrogen atom and Y is heterocyclic group represented by the formula (XV): 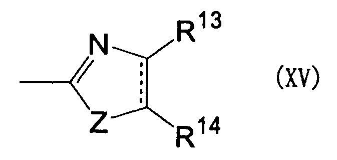 wherein a dotted line is a double bond or a single bond, Z is an oxygen atom or a sulfur atom, R¹³ and R¹⁴ are the same or different and each is a group corresponding to the substituent of a heterocyclic group, or optionally form, together with the adjacent carbon atom, a homocycle or heterocycle, optionally having substituent(s) (hereinafter to be also referred to as heterocyclic group(XV)) (hereinafter this step is to be also referred to as Step (e-2)).

When R⁶ is a hydrogen atom, R⁶ (hydrogen atom) of compound (VI) can be converted to heterocyclic group (XV) by the following Steps (e-2a) to (e-2e) according to the method described in Journal of Medicinal Chemistry, 44(8), 1286-1296 (2001).

That is, a compound represented by the formula (VIIIb) wherein Y is heterocyclic group (XV) (hereinafter to be also referred to as compound (VIIIb)) can be obtained as shown in the following reaction scheme,
Step (e-2a): a compound represented by the formula (VIa) wherein R⁶ is a hydrogen atom (hereinafter to be also referred to as compound (VIa)) or a salt thereof is converted to cyanohydrin represented by the formula (XVI) (hereinafter to be also referred to as cyanohydrin (XVI));
Step (e-2b): the obtained cyanohydrin (XVI) or a salt thereof is converted to imidate represented by the formula (XVIII) (hereinafter to be also referred to as imidate (XVIII)) or a salt thereof;
Step (e-2c): the obtained imidate (XVIII) or a salt thereof is reacted with a compound represented by the formula (XVa) (hereinafter to be also referred to as compound (XVa)) or a salt thereof to give a compound represented by the formula (XIX) (hereinafter to be also referred to as compound (XIX)) or a salt thereof; or
Step (e-2d): compound (VIa) or a salt thereof is reacted with a compound represented by the formula (XVb) (hereinafter to be also referred to as compound (XVb)) to give compound (XIX) or a salt thereof; and
Step (e-2e): the obtained compound (XIX) or a salt thereof is oxidized. 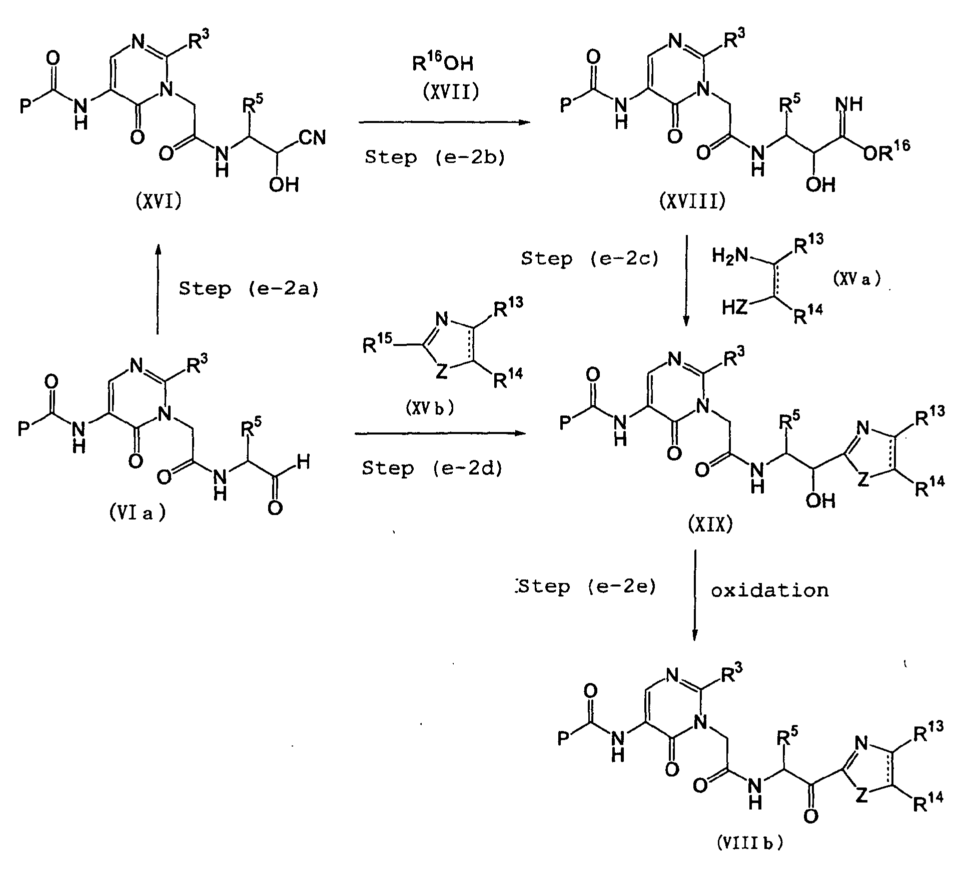 wherein R¹⁵ is a trialkylsilyl group, R¹⁶ is an alkyl group, and other symbols and the dotted line are as defined above.

The "group corresponding to the substituent of heterocyclic group" for R¹³ and R¹⁴ corresponds to the substituent of the "heterocyclic group optionally having substituent(s)" for Y. For example, a nitro group, a linear or branched chain alkoxy group (number of carbon: 1-6, e.g., methoxy group and the like), a halogen atom (e.g., chlorine atom, fluorine atom and the like), a linear or branched chain alkyl group (preferable number of carbon: 1-4, e.g., methyl group, ethyl group, propyl group and the like), an alkoxycarbonyl group (e.g., methoxycarbonyl group, ethoxycarbonyl group and the like) and the like can be mentioned.

As the homocyclic ring that may be formed by R¹³ and R¹⁴ in combination, for example, benzene, naphthalene, cyclohexane and the like can be mentioned.

As the heterocycle that may be formed by R¹³ and R¹⁴ in combination, for example, pyridine, piperidine and the like can be mentioned.

As the substituent that the homocycle and heterocycle may have, those similar to the above-mentioned "group corresponding to the substituent of heterocyclic group" can be mentioned.

The "trialkylsilyl group" for R¹⁵ is a silyl group substituted by the same or different alkyl group and, for example, trimethylsilyl group and the like can be mentioned.

The "alkyl group" for R¹⁶ is a linear or branched chain alkyl preferably having 1 to 3 carbon atoms, and, for example; methyl group, ethyl group, n-propyl group and the like can be mentioned, with preference given to methyl group and ethyl group.

### 5-2-1. Step (e-2a)

Step (e-2a) can be realized, for example, by reacting compound (VIa) with cyanohydrin and a base in a solvent.

The solvent usable in Step (e-2a) may be any as long as it does not inhibit the reaction and, for example, dichloromethane, THF, diethyl ether and the like can be mentioned, which may be used alone or in a combination of two or more kinds thereof. The amount of the solvent to be used is generally 5 to 50-fold weight, preferably 8 to 20-fold weight, relative to the compound (VIa).

As the cyanohydrins, for example, acetonecyanohydrin and the like can be mentioned. The amount of cyanohydrins to be used is generally 0.8-5 equivalents, preferably 1-3 equivalents, relative to the compound (VIa).

As the base, for example, triethylamine, pyridine, N-methylmorpholine and the like can be mentioned, with preference given to triethylamine. The amount of the base to be used is generally 0.5-3 equivalents, preferably 0.6-2 equivalents, relative to the compound (VIa).

Step (e-2a) is carried out within the temperature range of generally 0-50°C (preferably 10-30°C). The reaction completes within the above-mentioned temperature range generally for 30 min-24 hr (preferably 1 hr-8 hr).

Cyanohydrin (XVI) obtained in Step (e-2a) can be isolated and purified by a conventional method. For example, after the completion of reaction, the reaction mixture is washed with brine etc., partitioned and the obtained organic layer is concentrated to isolate cyanohydrin (XVI). Furthermore, cyanohydrin (XVI) can be purified by crystal precipitation, or subjecting the cyanohydrin to silica gel column chromatography, but the method is not limited to these. 5-2-2. Step (e-2b)

Step (e-2b) can be realized, for example, by reacting cyanohydrin (XVI) with alcohol represented by the formula (XVII): R¹⁶OH wherein R¹⁶ is as defined above (hereinafter to be also referred to as alcohol (XVII)) and hydrogen chloride in the presence or absence of a solvent.

The solvent usable in Step (e-2b) may be any as long as it does not inhibit the reaction, for example, chloroform, dichloromethane, THF and the like can be mentioned, which may be used alone or in a combination of two or more kinds thereof. When a solvent is used, the amount thereof to be used is generally 5 to 50-fold weight, preferably 8 to 20-fold weight, relative to the cyanohydrin (XVI).

As alcohol (XVII), methanol, ethanol and propanol can be specifically mentioned, with preference given to methanol and ethanol. The amount of alcohol (XVII) to be used is generally 0.1-50 equivalents, preferably 1-20 equivalents, relative to the cyanohydrin (XVI).

Hydrogen chloride may be introduced as a gas into the reaction system, or added as a solution of alcohol (XVII), or developed in the system by adding acetyl chloride, trimethylsilyl chloride and the like. The amount of hydrogen chloride to be used is generally 2-50 equivalents, preferably 5-30 equivalents, relative to the cyanohydrin (XVI).

Step (e-2b) is performed within the temperature range of generally -20°C to 30°C (preferably 0-20°C). The reaction completes within the above-mentioned temperature range generally for 30 min-24 hr (preferably 1 hr-16 hr).

Imidate (XVIII) obtained in Step (e-2b) can be isolated and purified by a conventional method, or subjected to the next step without particular purification.

### 5-2-3. Step (e-2c)

Step (e-2c) can be realized by, for example, reacting imidate (XVIII) with compound (XVa) in a solvent.

The solvent usable in Step (e-2c)) may be any as long as it does not inhibit the reaction and, for example, ethanol, acetonitrile, THF and the like can be mentioned, which may be used alone or in a combination of two or more kinds thereof. The amount of the solvent to be used is generally 5 to 50-fold weight, preferably 8 to 20-fold weight, relative to the imidate (XVIII).

As compound (XVa), 2-aminophenol, 2-aminothiophenol, 2-amino-3-hydroxypyridine, methyl 3-amino-4-hydroxybenzoate, aminoethanol and the like can be mentioned, with preference given to methyl 3-amino-4-hydroxybenzoate. The amount of compound (XVa) to be used is generally 0.8-2 equivalents, preferably 1-1.5 equivalents, relative to the imidate (XVIII).

In Step (e-2c), a base may be added to promote the reaction. As the base, for example, triethylamine, diisopropylethylamine, N-methylmorpholine and the like can be mentioned, with preference given to triethylamine. The amount of the base to be used is generally 0.5-5 equivalents, preferably 1-3 equivalents, relative to the imidate (XVIII).

Step (e-2c) is performed within the temperature range of generally from 0°C to the refluxing temperature of the solvent to be used (preferably 30°C-reflux). The reaction completes within the above-mentioned temperature range generally for 30 min-24 hr (preferably 1 hr-8 hr).

### 5-2-4. Step (e-2d)

Step (e-2d) can be realized by, for example, as shown in the following scheme, reacting compound (VIa) with compound (XVb) in a solvent to give a compound represented by the formula (XIX') (hereinafter to be also referred to as compound (XIX')) and eliminating silyl ether from the obtained compound (XIX'). 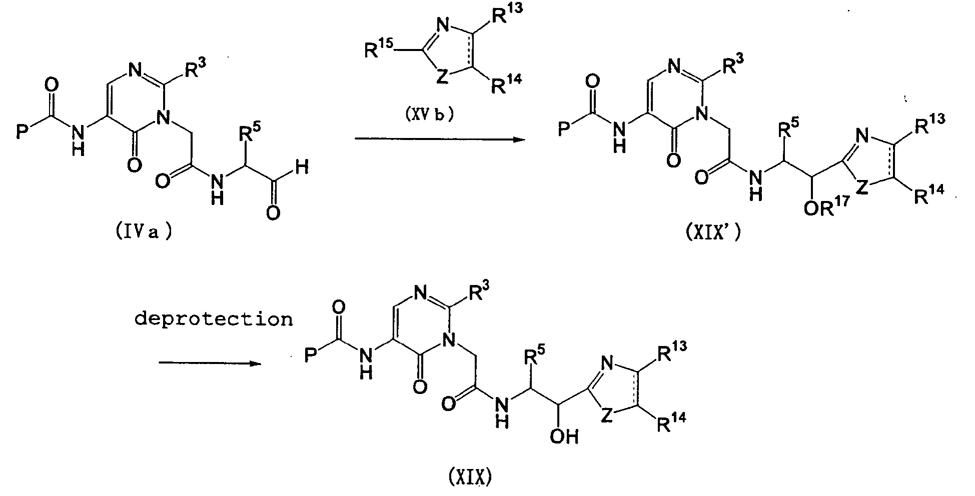 wherein each symbol and a dotted line are as defined above.

The solvent usable in Step (e-2d) may be any as long as it does not inhibit the reaction and, for example, dichloromethane, chloroform, THF and the like can be mentioned, which may be used alone or in a combination of two or more kinds thereof. The amount of the solvent to be used is generally 5 to 50-fold weight, preferably 8 to 20-fold weight, relative to the compound (VIa).

As compound (XVb), 2-trimethylsilylthiazole, 2-trimethylsilyloxazole and the like can be specifically mentioned, with preference given to 2-trimethylsilylthiazole. The amount of compound (XVb) to be used is generally 0.8-3 equivalents, preferably 1-1.5 equivalents, relative to the compound (VIa).

Compound (XVb) can be obtained by the method described in J. Org. Chem. 58, 3196 (1993), or a commercially available product may be used.

Step (e-2d) is performed within the temperature range of generally 0-50°C (preferably 10-30°C). The reaction completes within the above-mentioned temperature range generally for 30 min-24 hr (preferably 1 hr-8 hr).

The obtained compound (XIX') can be deprotected by adding an acid (e.g., hydrochloric acid, methanesulfonic acid etc.) or fluoride (e.g., tetra-n-butylammonium fluoride etc.) to the above-mentioned reaction mixture.

The amount of the acid or fluoride to be used is generally 1-10 equivalents, preferably 1-3 equivalents, relative to the compound (VIa).

The deprotection is carried out within the temperature range of generally 0-50°C (preferably 10-30°C). The reaction completes within the above-mentioned temperature range generally for 30 min-24 hr (preferably 30 min-8 hr).

Compound (XIX) obtained in Step (e-2c) or Step (e-2d) can be isolated and purified by a conventional method. For example, after the completion of the reaction, the reaction mixture is washed successively with aqueous acidic solution (e.g., hydrochloric acid, sulfuric acid and the like) and aqueous alkaline solution (e.g., saturated aqueous sodium hydrogen carbonate, brine and the like), partitioned and the obtained organic layer is concentrated to isolate compound (XIX). By further crystal precipitation or silica gel column chromatography, compound (XIX) can be purified, but the method is not limited to these.

### 5-2-5. Step (e-2e)

In Step (e-2e), compound (XIX) is oxidized by various known methods (e.g., Dess-Martin reagent, Swern oxidation, Pfitzner-Moffatt oxidation etc.) to give compound (IVb).

In the following, Pfitzner-Moffatt oxidation (J. Med. Chem., 30, 1617-1622(1987)), which is a preferable embodiment, is explained, though Step (e-2e) is not limited thereto.

The Pfitzner-Moffatt oxidization in Step (e-2e) can be realized, for example, by reacting compound (XIX) with a carbodiimide condensing agent and an acid in dimethyl sulfoxide and other solvent.

The solvent usable in the oxidization reaction may be any as long as it does not inhibit the reaction and, for example, toluene, chloroform, dichloromethane and the like can be mentioned, which may be used alone or in a combination of two or more kinds thereof. The amount of the solvent to be used is generally 5 to 50-fold weight, preferably 8 to 20-fold weight, relative to the compound (XIX).

The amount of dimethyl sulfoxide to be used is generally 2-100 equivalents, preferably 2-80 equivalents, relative to the compound (XIX).

As the carbodiimide condensing agent, for example, N,N'-dicyclohexylcarbodiimide (DCC), N-(3-dimethylaminopropyl)-N'-ethylcarbodiimide hydrochloride (EDC) and the like can be mentioned, with preference given to EDC. The amount of carbodiimide condensing agent to be used is generally 1-5 equivalents, preferably 1-3 equivalents, relative to the compound (XIX).

As the acid, for example, dichloroacetic acid, chloroacetic acid and the like can be mentioned, with preference given to dichloroacetic acid. The amount of the acid to be used is generally 0.8-10 equivalents, preferably 1-3 equivalents, relative to the compound (XIX).

The oxidization reaction is performed within the temperature range of generally 0-30°C (preferably 0-20°C). The reaction completes within the above-mentioned temperature range generally for 30 min-24 hr (preferably 1 hr-5 hr). 5-3. When R⁶ is hydrogen atom and Y is acyl group optionally having substituent(s) (hereinafter to be also referred to as Step (e-3))

When R⁶ is a hydrogen atom, R⁶ (hydrogen atom) of compound (VI) can be converted to an acyl group optionally having substituent(s) by the following Steps (e-3a)-(e-3g), according to the method described in WO98/09949.

That is, a compound represented by the formula (VIIIc) wherein Y is an acyl group optionally having substituent(s) (hereinafter to be also referred to as compound (VIIIc)) can be obtained as shown in the following reaction scheme,
Step (e-3a): compound (VIa) or a salt thereof is converted to cyanohydrin (XVI) or a salt thereof in the same manner as in Step (g-2a);
Step (e-3b): the obtained cyanohydrin (XVI) or a salt thereof is hydrolyzed to give a compound represented by the formula (XX) (hereinafter to be also referred to as compound (XX)) or a salt thereof;
Step (e-3c): the obtained compound (XX) or a salt thereof is reacted with N,O-dimethylhydroxylamine or a salt thereof to give a compound represented by the formula (XXI) (hereinafter to be also referred to as compound (XXI)) or a salt thereof; Step (e-3d): an oxazolidine ring is formed in the obtained compound (XXI) or a salt thereof to give a compound represented by the formula (XXII) (hereinafter to be also referred to as compound (XXII)) or a salt thereof;
Step (e-3e): the obtained compound (XXII) or a salt thereof is reacted with a reagent represented by the formula (XIVb) (hereinafter to be also referred to as reagent (XIVb)) to give a compound represented by the formula (XXIII) (hereinafter to be also referred to as compound (XXIII)) or a salt thereof;
Step (e-3f): the oxazolidine ring of the obtained compound (XXIII) or a salt thereof is opened to give a compound represented by the formula (XXIV) (hereinafter to be also referred to as compound (XXIV)) or a salt thereof; and
Step (e-3g): the obtained compound (XXIV) or a salt thereof is oxidized. 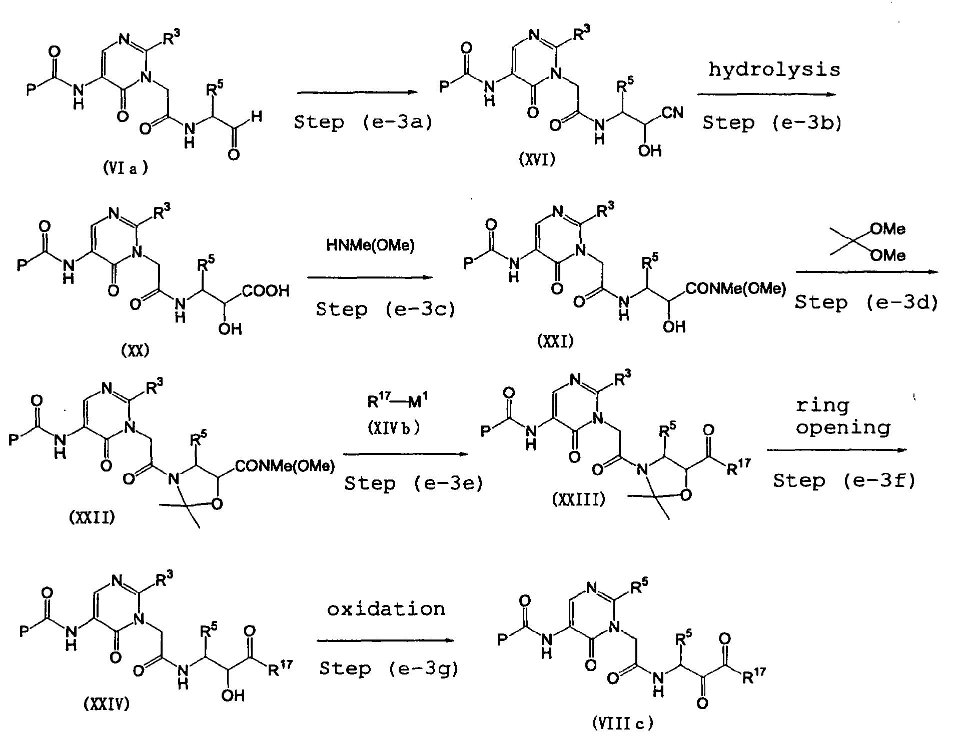 wherein R¹⁷ is a group formed by removing a carbonyl group from the acyl group optionally having substituent(s) for Y, and other symbols are as defined above.

As the "group formed by removing a carbonyl group from the acyl group optionally having substituent(s)" for R¹⁷, for example, 3-(2-pyridyloxy)propyl group and the like can be mentioned.

### 5-3-1. Step (e-3a)

In Step (e-3a), cyanohydrin (XVI) is obtained from compound (VIa) by a method similar to the method of Step (e-2a).

### 5-3-2. Step (e-3b)

Step (e-3b) can be realized by, for example, hydrolyzing cyanohydrin (XVI) with an acid in a solvent.

The solvent usable in Step (e-3b) may be any as long as it does not inhibit the reaction and, for example, 1,4-dioxane, THF, MTBE and the like can be mentioned, which may be used alone or in a combination of two or more kinds thereof. The amount of the solvent to be used is generally 5 to 50-fold weight, preferably 8 to 20-fold weight, relative to the cyanohydrin (XVI).

As the acid, for example, hydrochloric acid, sulfuric acid, p-toluenesulfonic acid and the like can be mentioned, with preference given to hydrochloric acid. The amount of the acid to be used is generally 3-100 equivalents, preferably 5-30 equivalents, relative to the cyanohydrin (XVI).

Step (e-3b) is performed generally within the temperature range of 0°C to the refluxing temperature of the solvent to be used (preferably 5-60°C). The reaction completes within the above-mentioned temperature range generally for 30 min-24 hr (preferably 1 hr-16 hr).

Compound (XX) obtained Step (e-3b) can be isolated and purified by a conventional method. For example, after the completion of reaction, the reaction mixture is washed with an aqueous acidic solution (e.g., hydrochloric acid, sulfuric acid and the like), partitioned and the obtained organic layer is concentrated to isolate compound (XX). Furthermore, compound (XX) can be purified by crystal precipitation or silica gel column chromatography, but the method is not limited to these. 5-3-3. Step (e-3c)

Step (e-3c) can be realized by, for example, condensing compound (XX) with N,O-dimethylhydroxylamine using a condensing agent in a solvent.

The solvent usable in Step (e-3c) may be any as long as it does not inhibit the reaction and, for example, dichloromethane, THF, diethyl ether and the like can be mentioned, which may be used alone or in a combination of two or more kinds thereof. The amount of the solvent to be used is generally 5 to 50-fold weight, preferably 8 to 20-fold weight, relative to the compound (XX).

As the condensing agent, for example, carbodiimide condensing agents (e.g., DCC, EDC etc.), chloroformates (e.g., methyl chloroformate, ethyl chloroformate, isobutyl chloroformate etc.) and the like can be mentioned.

The condensation reaction is carried out within the temperature range of generally -20°C to 20°C (preferably 0-10°C). The reaction completes within the above-mentioned temperature range generally for 15 min-16 hr (preferably 0.5 hr-2 hr).

Compound (XXI) obtained in Step (e-3c) can be isolated and purified by a conventional method. For example, after the completion of reaction, the reaction mixture is washed successively with aqueous acidic solution (e.g., hydrochloric acid, sulfuric acid and the like) and aqueous alkaline solution (e.g., saturated aqueous sodium hydrogen carbonate, brine and the like), partitioned and the obtained organic layer is concentrated to isolate compound (XXI). Furthermore, compound (XXI) can be purified by crystal precipitation or silica gel column chromatography, but the method is not limited to these. 5-3-4. Step (e-3d)

Step (e-3d) can be realized, for example, by reacting compound (XXI) with acetone dimethylacetal in a solvent or without solvent in the presence of an acid.

The solvent usable in Step (e-3d) may be any as long as it does not inhibit the reaction and, for example, acetone, ethyl acetate, THF and the like can be mentioned, which may be used alone or in a combination of two or more kinds thereof. The amount of the solvent to be used is generally 5 to 50-fold weight, preferably 8 to 20-fold weight, relative to the compound (XXI).

The amount of acetonedimethylacetal to be used is generally 1-5 equivalents, preferably 1-3 equivalents, relative to the compound (XXI).

As the acid, for example, p-toluenesulfonic acid, methanesulfonic acid, sulfuric acid and the like can be mentioned, with preference given to p-toluenesulfonic acid. The amount of the acid to be used is generally 0.1-3 equivalents, preferably 0.3-2 equivalents, relative to the compound (XXI).

Step (e-3d) is performed within the temperature range of generally -20°C to 40°C (preferably 0-20°C). The reaction completes within the above-mentioned temperature range generally for 30 min-24 hr (preferably 1 hr-16 hr).

Compound (XXII) obtained in Step (e-3d) can be isolated and purified by a conventional method. For example, after the completion of reaction, the reaction mixture is washed successively with aqueous acidic solution (e.g., hydrochloric acid, sulfuric acid and the like) and aqueous alkaline solution (e.g., saturated aqueous sodium hydrogen carbonate, brine and the like), partitioned and the obtained organic layer is concentrated to isolate compound (XXII). Furthermore, compound (XXII) can be purified by crystal precipitation or silica gel column chromatography, but the method is not limited to these. 5-3-5. Step (e-3e)

Step (e-3e) can be realized, for example, by reacting compound (XXII) with reagent (XIVb) in a solvent.

The solvent usable in Step (e-3e) may be any as long as it does not inhibit the reaction and, for example, ether solvents (e.g., THF, diethyl ether, MTBE etc.), halogen solvents (e.g., chloroform, dichloromethane etc.) and the like can be mentioned, which may be used alone or in a combination of two or more kinds thereof. The amount of the solvent to be used is generally 5 to 50-fold weight, preferably 8 to 20-fold weight, relative to the compound (XXII).

The reagent (XIVb) can be prepared from a compound represented by the formula R¹⁷-X wherein each symbol is as defined above by a method similar to the method for reagent (XIVa) in the aforementioned Step (e-1). For example, 3-(2-pyridyloxy)propylmagnesium bromide, 3-(2-pyridyloxy)propyl lithium and the like are preferable. The amount of the reagent (XIVb) to be used is, generally 1-5 equivalents, preferably 1-3 equivalents, relative to the compound (XXII).

Step (e-3e) is performed within the temperature range of generally -80°C to 40°C (preferably -78°C to 20°C). The reaction completes within the above-mentioned temperature range generally for 15 min-24 hr (preferably 1 hr-16 hr).

Compound (XXIII) obtained in step (e-3e) can be isolated and purified by a conventional method. For example, after the completion of reaction, the reaction mixture is washed successively with aqueous acidic solution (e.g., hydrochloric acid, sulfuric acid and the like) and aqueous alkaline solution (e.g., saturated aqueous sodium hydrogen carbonate, brine and the like), partitioned and the obtained organic layer is concentrated to isolate compound (XXIII). Furthermore, compound (XXIII) can be purified by crystal precipitation or silica gel column chromatography, but the method is not limited to these.

### 5-3-6. Step (e-3f)

Step (e-3f) can be realized, for example, by reacting compound (XXIII) with an acid in a solvent.

The solvent usable in Step (e-3f) may be any as long as it does not inhibit the reaction and, for example, water, methanol, ethanol and the like can be mentioned, which may be used alone or in a combination of two or more kinds thereof. The amount of the solvent to be used is generally 5 to 50-fold weight, preferably 8 to 20-fold weight, relative to the compound (XXIII).

As the acid, for example, hydrochloric acid, sulfuric acid, p-toluenesulfonic acid, methanesulfonic acid and the like can be mentioned, with preference given to p-toluenesulfonic acid and sulfuric acid. The amount of the acid to be used is generally 0.2-30 equivalents, preferably 1-20 equivalents, relative to the compound (XXIII).

Step (e-3f) is performed within the temperature range of generally from 20°C to the refluxing temperature of the solvent to be used. The reaction completes within the above-mentioned temperature range generally for 30 min-24 hr (preferably 3 hr-16 hr).

Compound (XXIV) obtained in step (e-3f) can be isolated and purified by a conventional method. For example, after the completion of reaction, the reaction mixture is washed successively with aqueous acidic solution (e.g., hydrochloric acid, sulfuric acid and the like) and aqueous alkaline solution (e.g., saturated aqueous sodium hydrogen carbonate, brine and the like), partitioned and the obtained organic layer is concentrated to isolate compound (XXIV). Furthermore, compound (XXIV) can be purified by crystal precipitation or silica gel column chromatography, but the method is not limited to these. 5-3-7. Step (e-3g)

In Step (e-3g), compound (XXIV) can be led to compound (VIIIc) by a method similar to the method of Step (e-2e) (e.g., Dess-Martin reagent, Swern oxidation, Pfitzner-Moffatt oxidation etc.).

### 5-4. When R⁶ is hydroxyl group and Y is trifluoromethyl group (hereinafter to be also referred to as step (e-4))

When R⁶ is a hydroxyl group, R⁶ (hydroxyl group) of compound (VI) can be converted to a trifluoromethyl group by the following Steps (g-4a)-(g-4d) according to the method described in Tetrahedron Lett., 36(42), 7761-7764 (1995).

A compound represented by the formula (VIIId) wherein Y is a trifluoromethyl group (hereinafter to be also referred to as compound (VIIId) can be obtained, as shown in the following scheme,

Step (e-4a): a compound represented by the formula (VIb) wherein R⁸ is a hydroxyl group (hereinafter to be also referred to as compound (VIb)) or a salt thereof is reacted with aldehyde represented by the formula (XXV): R¹⁸CHO wherein R¹⁸ is a hydrogen atom, an alkyl group (e.g., methyl group, ethyl group etc.), an aryl group optionally having substituent(s) (e.g., p-methoxyphenyl group etc.) and the like (hereinafter to be also referred to as aldehyde (XXV)) to give a compound represented by the formula (XXVI) (hereinafter to be also referred to as compound (XXVI)) or a salt thereof;

Step (e-4b): the obtained compound (XXVI) or a salt thereof is reacted with trimethyl(trifluoromethyl)silane to give a compound represented by the formula (XXVII) (hereinafter to be also referred to as compound (XXVII)) or a salt thereof;

Step (e-4c): a trimethylsilyl group of the obtained compound (XXVII) or a salt thereof is deprotected to give a compound represented by the formula (XXVIII) (hereinafter to be also referred to as compound (XXVIII)) or a salt thereof; and

Step (e-4d): an oxazolidine ring of the obtained compound (XXVIII) or a salt thereof is opened. 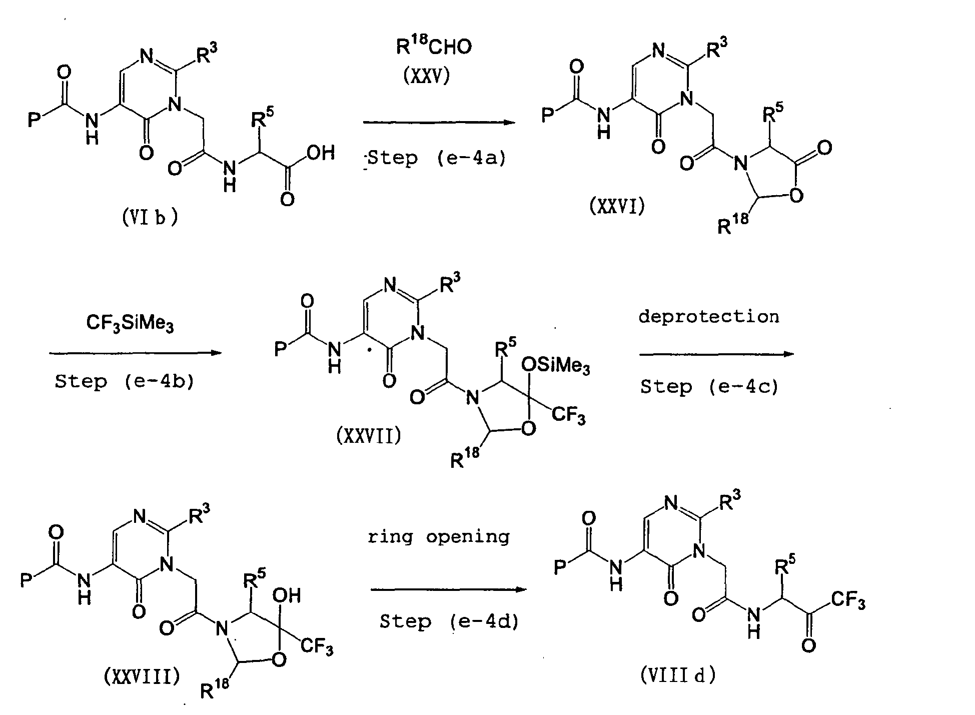 wherein each symbol is as defined above.

### 5-4-1. Step (e-4a)

Step (g-4a) can be realized by, for example, subjecting compound (VIb) and aldehyde (XXV) to a dehydration reaction in a solvent in the presence of an acid. The dehydration reaction can be performed, for example, by distilling water and the solvent from the mixture and returning only the solvent by an azeotropic reaction using a Dean-Stark tube.

As the solvent usable in Step (e-4a), for example, benzene, toluene, chloroform, diethyl ether, acetonitrile and the like can be mentioned, which may be used alone or in a combination of two or more kinds thereof. The amount of the solvent to be used is generally 5 to 50-fold weight, preferably 8 to 20-fold weight, relative to the compound (VIb).

As the acid, for example, p-toluenesulfonic acid, methanesulfonic acid and the like can be mentioned, with preference given to p-toluenesulfonic acid. The amount of the acid to be used is generally 0.01-5 equivalents, preferably 0.03-1 equivalent, relative to the compound (VIb).

As aldehyde (XXV), for example, paraformaldehyde, acetaldehyde and the like can be mentioned, with preference given to paraformaldehyde. The amount of aldehyde (XXV) to be used is generally 1-5 equivalents, preferably 1-3 equivalents, relative to compound (VIb).

Step (e-4a) is generally performed at the refluxing temperature of the solvent to be used. The reaction completes within the above-mentioned temperature range, generally 30 min-24 hr (preferably 1 hr-16 hr).

Compound (XXVI) obtained in Step (e-4a) can be isolated and purified by a conventional method. After the completion of the reaction, for example, the reaction mixture is washed with an aqueous acidic solution (e.g., hydrochloric acid, sulfuric acid etc.), and partitioned, and the obtained organic layer is concentrated to isolate compound (XXVI). Furthermore, crystal precipitation may be performed, or the reaction mixture is subjected to silica gel column chromatography to purify compound (XXVI), but the method is not limited to these.

### 5-4-2. Step (e-4b)

Step (e-4b) can be realized by, for example, reacting compound (XXVI) with trimethyl(trifluoromethyl)silane in a solvent in the presence of a fluoride salt. In this case, ultrasonication is preferably applied to the reaction system to promote the reaction.

The solvent usable in Step (e-4b) may be any as long as it does not inhibit the reaction, and, for example, THF, MTBE and the like can be mentioned. Two or more kinds thereof may be used in combination. The amount of solvent to be used is generally 5- to 50-fold weight, preferably 8- to 20-fold weight, relative to compound (XXVI).

As the fluoride salt, for example, cesium fluoride, tetra-n-butylammonium fluoride and the like can be mentioned, with preference given to cesium fluoride. The amount of the fluoride salt to be used can be a catalyst amount, which is generally 1-5 equivalents, preferably 1-2 equivalents, relative to compound (XXVI).

The amount of trimethyl(trifluoromethyl)silane to be used is generally 0.8-5 equivalents, preferably 1-2 equivalents, relative to compound (XXVI).

Step (e-4b) is performed within the range of generally 0°C to 80°C (preferably 5°C to 40°C). The reaction completes within the above-mentioned temperature range, generally 30 min-24 hr (preferably 1 hr-16 hr).

Compound (XXVII) obtained in Step (e-4b) can be isolated and purified by a conventional method. After the completion of the reaction, for example, the reaction mixture is washed with an aqueous acidic solution (e.g., hydrochloric acid, sulfuric acid etc.), and partitioned, and the obtained organic layer is concentrated to isolate compound (XXVII). Furthermore, crystal precipitation may be performed, or the reaction mixture is subjected to silica gel column chromatography to purify compound (XXVII), but the method is not limited to these.

### 5-4-3. Step (e-4c)

Step (e-4c) can be realized by, for example, treating compound (XXVII) with a fluoride salt in a solvent. In this case, ultrasonication is preferably applied to the reaction system to promote the reaction.

The solvent usable in Step (e-4c) may be any as long as it does not inhibit the reaction, and, for example, THF, MTBE, dichloromethane, chloroform and the like can be mentioned. Two or more kinds thereof may be used in combination. The amount of solvent to be used is generally 5- to 50-fold weight, preferably 8-to 12-fold weight, relative to compound (XXVII).

As the fluoride salt, for example, cesium fluoride, tetra-n-butylammonium fluoride and the like can be mentioned, with preference given to cesium fluoride. The amount of the fluoride salt to be used is generally 1-5 equivalents, preferably 1-2 equivalents, relative to compound (XXVII).

Step (e-4c) is performed within the temperature range of generally 0°C to 80°C (preferably 0°C to 40°C). The reaction completes within the above-mentioned temperature range generally for 30 min-24 hr (preferably 1 hr-16 hr).

Compound (XXVIII) obtained in Step (e-4c) can be isolated and purified by a conventional method. After the completion of the reaction, for example, the reaction mixture is washed with an aqueous acidic solution (e.g., hydrochloric acid, sulfuric acid etc.), and partitioned, and the obtained organic layer is concentrated to isolate compound (XXVIII). Furthermore, crystal precipitation may be performed, or the reaction mixture is subjected to silica gel column chromatography to purify compound (XXVIII), but the method is not limited to these.

### 5-4-4. Step (e-4d)

Step (e-4d) can be realized by, for example, treating compound (XXVIII) with an acid in a solvent.

The solvent usable in Step (e-4d) may be any as long as it does not inhibit the reaction, and, for example, acetonitrile, THF, ethyl acetate, chloroform and the like can be mentioned. Two or more kinds thereof may be used in combination. The amount of solvent to be used is generally 5-to 50-fold weight, preferably 8- to 20-fold weight, relative to compound (XXVIII).

As the acid, for example, strong acidic ion exchange resins (e.g., Amberlite (trademark) R-120, Amberlyst (trademark) etc.) and the like can be mentioned, with preference given to strong acidic ion exchange resin. The amount of the acid to be used is generally 0.1- to 100-fold weight, preferably 0.5- to 20-fold weight, relative to compound (XXVIII).

Step (e-4d) is performed within the range of generally 0°C to 80°C (preferably 20°C to 50°C). The reaction completes within the above-mentioned temperature range, generally 30 min-24 hr (preferably 1 hr-16 hr).

### 5-5. When R⁶ is hydrogen atom and Y is trifluoromethyl group (hereinafter to be also referred to as Step (e-5))

When R⁶ is a hydrogen atom, R⁶ (hydrogen atom) of compound (VI) can be converted to a trifluoromethyl group by the following Steps (e-5a)-(e-5c) according to the method described in Tetrahedron Lett., 36(42), 7761-7764 (1995).

That is, compound (VIIId) can be obtained as shown in the following scheme,
Step (e-5a): compound (VIa) or a salt thereof is reacted with trimethyl(trifluoromethyl)silane to give a compound represented by the formula (XXIX) or a salt thereof;
Step (e-5b): a trimethylsilyl group of the obtained compound represented by the formula (XXIX) or a salt thereof is eliminated to give a compound represented by the formula (XXX) or a salt thereof;
Step (e-5c): the obtained compound represented by the formula (XXX) or a salt thereof is oxidized. 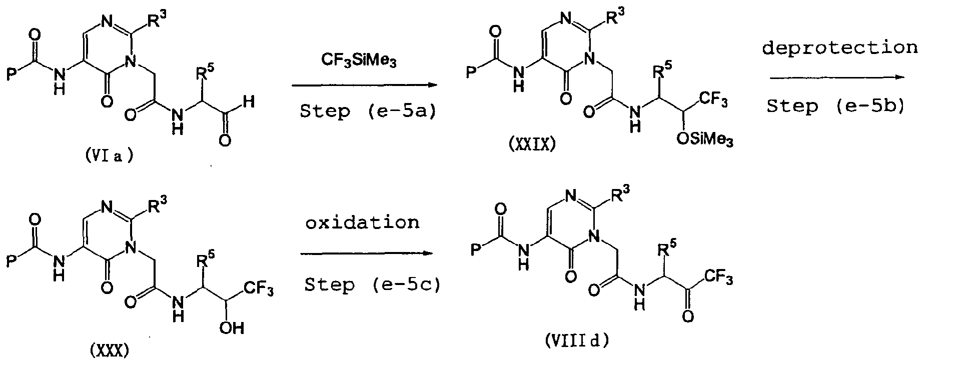 wherein each symbol is as defined above.
Step (e-5a) can be performed according to a method and conditions similar to those of Step (e-4b), Step (e-5b) can be performed according to a method and conditions similar to those of Step (e-4c), and Step (e-5c) can be performed according to a method and conditions similar to those of Step (e-2e).
   Compound (VIII) obtained by Steps (e-1)-(e-5) can be isolated and purified by a conventional method as mentioned above. After the completion of the reaction, for example, the reaction mixture is washed successively with an aqueous acidic solution (e.g., hydrochloric acid, sulfuric acid etc.) or an aqueous alkali solution (e.g., saturated aqueous sodium hydrogen carbonate, brine etc.) and partitioned, and the obtained organic layer is concentrated to isolate compound (VIII). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), acetates (e.g., ethyl acetate, butyl acetate etc.), water or a mixed solvent thereof etc.] is added to allow crystal precipitation, or the reaction mixture is subjected to silica gel column chromatography to purify compound (VIII), but the method is not limited to these.

### 6. Step (f)

In Step (f), when R⁴ is other than a hydrogen atom, compound (III) or a salt thereof is converted to a carboxylic acid form, and the carboxylic acid form is condensed with compound (IX) to give compound (VIII) or a salt thereof.

Step (f) is advantageous in that compound (VIII) can be produced by directly condensing compound (III) with compound (IX) in a short step.

When R⁴ of compound (III) is other than a hydrogen atom, namely, an alkyl group or an aralkyl group, a carboxylic acid form is first obtained by a method similar to that in Step (b).

A carboxylic acid form and compound (IX) are condensed by the method of Step (b) used for condensing a carboxylic acid form with N-hydroxysuccinimide, wherein compound (IX) or a salt thereof is used instead of N-hydroxysuccinimide under similar conditions.

In a preferable embodiment, moreover, a carboxylic acid form is activated with an activating agent in a solvent and condensed with compound (IX) or a salt thereof to give compound (VIII).

This embodiment is explained below but Step (f) is not limited by this embodiment.

As the activating agent, those similar to ones used in Step (b) can be mentioned, with preference given to chloroformates, particularly, ethyl chloroformate, methyl chloroformate, isobutyl chloroformate and the like.

The amount of the activating agent to be used is generally 0.9-1.5 equivalents, preferably 1-1.2 equivalents, relative to a carboxylic acid form.

During activation of a carboxylic acid form, the base may be added as necessary. As such base, a base similar to that used in Step (b) can be mentioned, with preference given to N-methylmorpholine, triethylamine and pyridine.

The amount of the base to be used is generally 0.9-2 equivalents, preferably 1-1.3 equivalents, relative to a carboxylic acid form.

The solvent to be used for activation of carboxylic acid form may be any as long as it does not inhibit the reaction, and, for example, acetates (e.g., ethyl acetate, isopropyl acetate, isobutyl acetate, n-butyl acetate etc.), hydrocarbons (e.g., toluene, benzene, xylene etc.), acetonitrile, THF, halogen solvents (e.g., dichloromethane, dichloroethane etc.) and the like can be mentioned. These may be used in combination of two or more kinds thereof, and a single or mixed solvent of acetonitrile, THF and the like is more preferable. The amount of the solvent to be used is generally 3- to 50-fold weight, preferably 5- to 20-fold weight, relative to a carboxylic acid form.

The activation of the carboxylic acid compound is performed within the temperature range of generally -30°C to 40°C (preferably 0°C to 30°C). The activation completes within the above-mentioned temperature range generally for 5 min-5 hr (preferably 10 min-2 hr).

After activation of a carboxylic acid form, compound (IX) is added to the reaction mixture. The order of addition may be reverse or simultaneous.

The amount of compound (IX) to be used is generally 0.9-2 equivalents, preferably 1-1.2 equivalents, relative to a carboxylic acid form.

When compound (IX) is in the form of a salt, it is preferable to concurrently add, for neutralization, a base similar to the base used for activation of a carboxylic acid form.

The amount of the base to be used is generally 0.9-2 equivalents, preferably 1-1.2 equivalents, relative to compound (IX).

Condensation with compound (IX) is performed within the range of generally -30°C to 40°C (preferably -20°C to 30°C). The condensation completes within the above-mentioned temperature range generally for 0.5 hr-5 hr (preferably 1 hr-3 hr).

Compound (VIII) can be isolated and purified by a conventional method. After the completion of the reaction, for example, the reaction mixture is washed successively with an aqueous acidic solution (e.g., hydrochloric acid, sulfuric acid etc.) or an aqueous alkali solution (e.g., saturated aqueous sodium hydrogen carbonate, brine etc.) and partitioned, and the obtained organic layer is concentrated to isolate compound (VIII). Furthermore, a crystal precipitation solvent [e.g., ethers (e.g., diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] is added to allow crystal precipitation, or the reaction mixture is subjected to silica gel column chromatography to purify compound (VIII), but the method is not limited to these.

Compound (IX), which is a starting material of Step (f) can be synthesized using compound (V) as a starting material by protecting an amino group with a benzyloxycarbonyl group, a tert-butoxycarbonyl group and the like by a known method, converting a group represented by R⁶ to a group represented by Y by a method similar to that in the above-mentioned Step (e) and deprotecting by a known method.

### 7. Step (g)

In Step (g), the 5-position P-C(=O)-, wherein P is as defined above, of pyrimidine ring of compound (VI) is deprotected to give compound (X) or a salt thereof.

For the deprotection, a method known to those of ordinary skill in the art, such as the amide-deprotecting method described in Protective Groups in Organic Synthesis, 2nd ed., A Wiley Interscience Publication (1991), can be applied without limitation. To avoid side reactions such as decomposition and the like, mild conditions are preferable.

As a preferable embodiment of Step (g), (1) deprotection by enzyme reaction [hereafter to be also referred to as Step (g-1)] and (2) deprotection under acidic conditions [hereafter to be also referred to as Step (g-2)] can be mentioned. Step (g-1) and step (g-2) are specifically explained in the following, but Step (g) is not limited to these embodiments.

### 7-1. Step (g-1)

In Step (g-1), compound (VII) can be produced by, for example, reacting compound (VI) with an enzyme in a solvent to achieve deprotection.

The enzyme to be used in Step (g-1) is not particularly limited as long as it can deprotect the 5-position P-C(=O)-, wherein P is as defined above, of the pyrimidine ring, and conventionally known enzymes derived from microorganisms such as bacteria, actinomycetes, fungi and the like, or animals or plants, such as penicillin amidase, penicillin acylase and the like, can be mentioned.

It is also preferable to use an enzyme immobilized on a resin and the like because it facilitates separation from a compound and recycled use. As these enzymes and immobilized enzymes, commercially available ones can be used.

The amount of the enzyme or a solution obtained by treating mold only needs to be an amount capable of showing the object effect (effective amount) and the effective amount can be easily determined by those of ordinary skill in the art through a simple preliminary test. In the case of, for example, a commercially available enzyme solution, it is 0.01 ml-2 ml per 1 g of compound (VI) to be a substrate.

While the protecting group of compound (VI), which is a starting material, can be appropriately determined based on the substrate characteristics of the enzyme to be used. When it is a preferable enzyme, penicillin amidase, a phenylacetyl group wherein P is benzyl is preferable.

As a solvent usable in Step (g-1), water or a mixture of hydrophilic solvent to the degree the enzyme activity is not impaired can be used, and use of water alone is preferable. As the hydrophilic solvent, one or more kinds of N,N-dimethylformamide, dimethyl sulfoxide, THF, acetone and the like can be used. The amount of the solvent to be used is generally 5- to 200-fold weight, preferably 10- to 100-fold weight, relative to compound (VI).

The solvent is preferably adjusted to an optimal pH for an enzyme (e.g., about pH 6-8) with a buffer component, such as potassium dihydrogen phosphate, dipotassium hydrogen phosphate and the like.

While the concentration of a buffer component in a solvent is not particularly limited as long as it does not inhibit the enzyme reaction, it is about 0.001M - 0.2M. It is also possible to adjust pH by appropriately adding a base and the like during the reaction.

In Step (g-1), a surfactant may be added as necessary to promote the reaction. As such surfactant, for example, Triton X-100 (trademark), Tween (trademark) and the like can be mentioned, with preference given to Triton X-100. The amount of the surfactant to be used is generally 0.001- to 0.05-fold weight, preferably 0.01- to 0.05-fold weight, relative to compound (VI).

Step (g-1) is performed within the range of generally 20°C to 50°C (preferably 30°C to 40°C). The reaction completes within the above-mentioned temperature range, generally 30 min-1 week (preferably 1 hr-20 hr).

After the completion of the reaction, the obtained compound (VII) can be separated from an enzyme by a conventional method. In the case of an immobilized enzyme, for example, a soluble solvent of compound (VII) (e.g., acetonitrile etc.) is added as necessary, and the mixture is filtered to separate compound (VII) in the filtrate. In the case of an unimmobilized enzyme, water is added to the reaction mixture as necessary to allow precipitation of compound (VII), which is collected by filtration to separate compound (VII) as a filtered substance, but the method is not limited to these methods.

The separated compound (VII) can be purified by adding a solvent for crystal precipitation (e.g., ethers (e.g., diethyl ether, THF and the like), acetone, acetonitrile, hydrocarbon solvents (e.g., toluene, benzene, hexane, heptane and the like), halogen solvents (e.g., dichloromethane, dichloroethane and the like), alcohols (e.g., methanol, ethanol, isopropanol and the like), water, a mixed solvent thereof and the like) and the like to allow crystal precipitation, or subjecting the compound to silica gel column chromatography, but the method is not limited to these.

### 7-2. Step (g-2)

In Step (g-2), compound (VII) can be produced by, for example, adding an acid to compound (VI) in a solvent to allow deprotection. The order of addition may be reverse or simultaneous.

The solvent usable in Step (g-2) may be any as long as it does not inhibit the reaction. When an acid is used, at least a protic solvent such as water, alcohol and the like needs to be present. As the solvent, for example, acetates (e.g., ethyl acetate, isopropyl acetate, isobutyl acetate, n-butyl acetate etc.), hydrocarbons (e.g., toluene, benzene, xylene etc.), acetonitrile, THF, alcohols (e.g., methanol, ethanol, isopropyl alcohol, n-butanol etc.), N,N-dimethylformamide and the like can be mentioned. These may be used in combination of two or more kinds thereof and methanol, ethanol, acetonitrile, isopropyl alcohol, water and the like are preferable, and a single or mixed solvent of methanol, acetonitrile, water and the like is particularly preferable. The amount of the solvent to be used is generally 3- to 50-fold weight, preferably 5- to 20-fold weight, relative to compound (VI).

As an acid to be used in Step (g-2), inorganic acids (e.g., hydrogen chloride, sulfuric acid etc.), organic acids (e.g., methanesulfonic acid, p-toluenesulfonic acid, trifluoroacetic acid etc.) and the like can be mentioned, with preference given to hydrogen chloride and the like.

The amount of the acid to be used is generally 1-50 equivalent, preferably 3-10 equivalent, relative to the compound (VI).

The acid is preferably used in the form of a solution of the solvent to be used, wherein a solvent and an acid are added such that the amount thereof fall within the above-mentioned ranges.

Step (g-2) is performed within the temperature range of generally 0°C to the refluxing temperature of the solvent to be used (preferably 20-60°C). The reaction completes within the above-mentioned temperature range generally for 60 min-overnight (preferably 1 hr-5 hr).

After the completion of the reaction, compound (VII) is present in the reaction mixture in the form of a salt with the acid used. Thus, the acid addition salt can be isolated by concentrating the reaction mixture. The isolated acid addition salt of compound (VII) can be purified by adding a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] thereto to allow crystal precipitation.

For isolation of a free form of compound (VII), alkali addition in a protic solvent such as water, alcohol and the like is applied, or, after the completion of the reaction, the reaction mixture is washed with an aqueous alkali solution (e.g., saturated aqueous sodium hydrogen carbonate, brine etc.) and partitioned, and the obtained organic layer is concentrated, whereby compound (VII) can be isolated. Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like are added to allow crystal precipitation, or the reaction mixture is subjected to silica gel column chromatography to purify a free form of compound (VII), but the method is not limited to these.

Compound (VII) produced in Step (g) is a novel compound and is useful as a highly versatile synthetic intermediate for producing compound (XII) and other enzyme inhibitors (J. Med. Chem., 2001, 44, 1286-1296).

For example, compound (XII) can be produced by Steps (h), (i) and (j) explained in the following.

### 8. Step (h)

In Step (h), compound (X) or a salt thereof can be obtained by protection of an amino group of compound (VII) with a group other than an acyl group by a known method, such as the methods recited on page 309 ff. of Protective Groups in Organic Synthesis, 2nd edn., A Wiley Interscience Publication, 1991.

Protection with a Cbz group, which is a preferable embodiment of Step (h), is explained in the following, which is not to be construed as limiting Step (h).

Protection with a Cbz group can be realized by, for example, reacting compound (VII) with a benzyloxycarbonylation agent (hereinafter to be also referred to as Cbz agent) in a solvent.

The solvent usable in the protection reaction may be any as long as it does not inhibit the reaction, and, for example; water, acetonitrile, ethyl acetate, THF, chloroform and the like can be mentioned. Two or more kinds thereof may be used in combination. The amount of the solvent to be used is generally 5- to 50-fold weight, preferably 8- to 20-fold weight, relative to compound (VII).

As the Cbz agent, for example, benzyloxycarbonyl chloride and the like can be mentioned. The amount of the Cbz agent to be used is generally 0.9-2 equivalents, preferably 1-1.3 equivalents, relative to compound (VII).

In the protection reaction, a base (e.g., sodium hydroxide, triethylamine, pyridine, sodium carbonate etc.) may be added. The amount of the base to be used is generally 0.9-3 equivalents, preferably 1-1.5 equivalents, relative to compound (VII).

The protection reaction is performed within the range of generally -20°C to 40°C (preferably 0°C to 20°C). The reaction completes within the above-mentioned temperature range, generally 30 min-24 hr (preferably 1 hr-16 hr).

The obtained compound (X) can be isolated and purified by a conventional method. After the completion of the reaction, for example, the reaction mixture is washed successively with an aqueous acidic solution (e.g., hydrochloric acid, sulfuric acid etc.) or an aqueous alkali solution (e.g., saturated aqueous sodium hydrogen carbonate, brine etc.) and partitioned, and the obtained organic layer is concentrated to isolate compound (X). Furthermore, crystal precipitation may be performed, or the reaction mixture is subjected to silica gel column chromatography to purify compound (X), but the method is not limited to these.

### 9. Step (i)

In Step (i), a group represented by R⁶ of compound (X) or a salt thereof is converted to a group represented by Y to give compound (XI) or a salt thereof.

Step (i) can be performed by a method and reaction conditions similar to those in the above-mentioned Step (g).

### 10. Step (j)

In Step (j), a protecting group Q of compound (XI) or a salt thereof is eliminated by a known method such as the methods recited in page 309 ff. of Protective Groups in Organic Synthesis, 2nd edn., A Wiley Interscience Publication, 1991 to give compound (XII) or a salt thereof.

Deprotection of a compound wherein Q is a Cbz group, which is a preferable embodiment of Step (j), is explained in the following, which is not to be construed as limiting Step (j).

Deprotection of Cbz group can be realized by, for example, hydrogenolysis of compound (XI) in a solvent.

The solvent usable in the hydrogenolysis may be any as long as it does not inhibit the reaction, and, for example, ethanol, methanol, isopropanol, ethyl acetate, THF and the like can be mentioned. Two or more kinds thereof may be used in combination. The amount of solvent to be used is generally 5-to 50-fold weight, preferably 8- to 20-fold weight, relative to compound (XI).

As the catalyst to be used for the hydrogenolysis, for example, palladium on carbon, palladium hydroxide and the like can be mentioned. The amount of the catalyst to be used is generally 0.01- to 0.5-fold weight, preferably 0.05- to 0.2-fold weight, relative to compound (XI).

In the hydrogenolysis, an acid (e.g., hydrogen chloride, methanesulfonic acid etc.) may be added to promote the reaction. The amount of the acid to be used is generally 0.05-1.5 equivalents, preferably 0.1-1 equivalent, relative to compound (XI).

The reaction is carried out within the temperature range of generally 10°C to 80°C (preferably 20°C to 50°C). The reaction completes within the above-mentioned temperature range generally for 30 min-24 hr (preferably 1 hr-16 hr).

The obtained compound (XII) can be isolated and purified by a conventional method. After the completion of the reaction, for example, the catalyst is removed by filtration and the like, and alkali addition is applied in a protic solvent such as water, alcohol and the like, or the reaction mixture is washed with an aqueous alkali solution (e.g., saturated aqueous sodium hydrogen carbonate, brine etc.), and partitioned, and the obtained organic layer is concentrated to isolate compound (XII). Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like is added to allow crystal precipitation, or the reaction mixture is subjected to silica gel column chromatography to purify a free form of compound (XII), but the method is not limited to these. 11. Step (k)

In Step (k), the 5-position P-C(=O)-, wherein P is as defined above, of a pyrimidine ring of compound (VIII) is eliminated to give compound (XII) or a salt thereof.

For the deprotection, a method known to those of ordinary skill in the art, such as the amide-deprotection method described in Protective Groups in Organic Synthesis, 2nd edn., A Wiley Interscience Publication, 1991, can be applied without limitation. To avoid side reactions, such as decomposition and the like, the reaction is preferably performed under mild conditions, such as the conditions in the above-mentioned Step (g).

As preferable embodiments of Step (k), (1) deprotection by enzyme reaction [hereinafter to be also referred to as Step (k-1)] and (2) deprotection under acidic conditions [hereinafter to be also referred to as Step (k-2)] can be mentioned as in Step (g). While Step (k-1) and Step (k-2) are specifically explained in the following but Step (k) is not limited to these embodiments.

### 11-1. Step (k-1)

In Step (k-1), compound (XII) can be produced by, for example, reacting compound (VIII) and an enzyme in a solvent to allow deprotection.

As the enzyme to be used for Step (k-1), those similar as in Step (g-1) can be mentioned, with preference given to penicillin amidase, penicillin acylase and the like.

As an enzyme, those immobilized on a resin and the like are preferably used, as in Step (g-1).

The amount of the enzyme or a solution obtained by treating mold to be used only need to be in an amount capable of showing the object effect (effective amount) and the effective amount can be easily determined by those of ordinary skill in the art through a simple preliminary test. In the case of, for example, a commercially available enzyme solution, it is 0.01 ml-2 ml per 1 g of compound (VIII) to be a substrate.

While the protecting group of compound (VIII), which is a starting material, can be appropriately determined based on the substrate characteristics of the enzyme to be used. When it is a preferable enzyme, penicillin amidase, a phenylacetyl group wherein P is benzyl is preferable.

As a solvent usable in Step (k-1), water or a mixture of hydrophilic solvent to the degree the enzyme activity is not impaired can be used, and use of water alone is preferable. As the hydrophilic solvent, one or more kinds of dimethyl sulfoxide, acetone and the like can be used. The amount of the solvent to be used is generally 5- to 200-fold weight, preferably 10- to 100-fold weight, relative to compound (VIII).

The solvent is preferably adjusted to an optimal pH for an enzyme (e.g., about pH 6-8) with a buffer component, such as potassium dihydrogen phosphate, dipotassium hydrogen phosphate and the like.

While the concentration of a buffer component in a solvent is not particularly limited as long as it does not inhibit the enzyme reaction, it is about 0.001M - 0.2M.

It is also possible to adjust pH by appropriately adding a base and the like during the reaction.

In Step (k-1), a surfactant may be added as necessary to promote the reaction. As such surfactant, for example, Triton X-100 (trademark), Tween (trademark) and the like can be mentioned, with preference given to Triton X-100. The amount of the surfactant to be used is generally 0.001- to 0.05-fold weight, preferably 0.01- to 0.05-fold weight, relative to compound (VIII).

Step (k-1) is performed within the range of generally 20°C to 50°C (preferably 30°C to 40°C). The reaction completes within the above-mentioned temperature range, generally 30 min-1 week (preferably 1 hr-20 hr).

After the completion of the reaction, the obtained compound (XII) can be separated from an enzyme by a conventional method. In the case of an immobilized enzyme, for example, a soluble solvent of compound (XII) (e.g., acetonitrile etc.) is added as necessary, and the mixture is filtered to separate compound (XII) in the filtrate. In the case of an unimmobilized enzyme, water is added to the reaction mixture as necessary to allow precipitation of compound (XII), which is collected by filtration to separate compound (XII) as a filtered substance, but the method is not limited to these methods.

The separated compound (XII) can be purified, for example, by adding a solvent for crystal precipitation (e.g., ethers (e.g., diethyl ether, THF and the like), acetone, acetonitrile, hydrocarbon solvents (e.g., toluene, benzene, hexane, heptane and the like), halogen solvents (e.g., dichloromethane, dichloroethane and the like), alcohols (e.g., methanol, ethanol, isopropanol and the like), water, a mixed solvent thereof and the like) and the like to allow crystal precipitation, or subjecting the compound to silica gel column chromatography, but the method is not limited to these.

### 11-2. Step (k-2)

In Step (k-2), compound (XII) can be produced by, for example, adding an acid to compound (VIII) in a solvent to allow deprotection. The order of addition may be reverse or simultaneous.

The solvent usable in Step (k-2) may be any as long as it does not inhibit the reaction. When an acid is used, at least a protic solvent such as water, alcohol and the like needs to be present. As the solvent, for example, acetates (e.g., ethyl acetate, isopropyl acetate, isobutyl acetate, n-butyl acetate etc.), hydrocarbons (e.g., toluene, benzene, xylene etc.), acetonitrile, THF, alcohols (e.g., methanol, ethanol, isopropyl alcohol, n-butanol etc.), N,N-dimethylformamide and the like can be mentioned. These may be used in combination of two or more kinds thereof and methanol, ethanol, acetonitrile, isopropyl alcohol, water and the like are preferable, and a single or mixed solvent of methanol, acetonitrile, water and the like is particularly preferable. The amount of the solvent to be used is generally 3- to 50-fold weight, preferably 5- to 20-fold weight, relative to compound (VIII).

As an acid to be used in Step (k-2), inorganic acids (e.g., hydrogen chloride, sulfuric acid etc.), organic acids (e.g., methanesulfonic acid, p-toluenesulfonic acid, trifluoroacetic acid etc.) and the like can be mentioned, with preference given to hydrogen chloride and the like.

The amount of the acid to be used is generally 1-50 equivalents, preferably 3-10 equivalents, relative to compound (VIII).

The acid is preferably used in the form of a solution of the solvent to be used, wherein a solvent and an acid are added such that the amount thereof fall within the above-mentioned ranges.

Step (k-2) is performed within the range of generally from 0°C to the refluxing temperature of the solvent to be used (preferably 20°C to 60°C). The reaction completes within the above-mentioned temperature range, generally 60 min-one night (preferably 1 hr-5 hr).

After the completion of the reaction, compound (XII) is present in the reaction mixture in the form of a salt with the acid used. Thus, the acid addition salt can be isolated by concentrating the reaction mixture. The isolated acid addition salt of compound (XII) can be purified by adding a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] thereto to allow crystal precipitation.

For isolation of a free form of compound (XII), alkali addition in a protic solvent such as water, alcohol and the like is applied, or, after the completion of the reaction, the reaction mixture is washed with an aqueous alkali solution (e.g., saturated aqueous sodium hydrogen carbonate, brine etc.) and partitioned, and the obtained organic layer is concentrated, whereby compound (XII) can be purified. Furthermore, a crystal precipitation solvent [e.g., ethers (e.g.: diethyl ether, THF etc.), acetone, acetonitrile, hydrocarbon solvents (e.g., toluene, benzene, hexane, heptane etc.), halogen solvents (e.g., dichloromethane, dichloroethane etc.), alcohols (e.g., methanol, ethanol, isopropanol etc.), water or a mixed solvent thereof etc.] and the like are added to allow crystal precipitation, or the reaction mixture is subjected to silica gel column chromatography to purify a free form of compound (XII), but the method is not limited to these.

### Examples

The present invention is explained in more detail by referring to Examples, which are not to be construed as limitative.

### Reference Example 1

### 4-(N,N-dimethylaminomethylene)-2-methyl-5-oxazolinone

To N-acetylglycine (20.0 g, 171 mmol) were added phosphorus oxychloride (67.0 g, 437 mmol) and N,N-dimethylformamide (33.0 g, 451 mmol) in an ice bath, and the mixture was stirred at 45°C for 1.5 hrs. The reaction mixture was concentrated under reduced pressure, and added dropwise to 28% aqueous ammonia (150 ml) while maintaining not more than 10°C. The mixture was stirred in an ice bath for 1 hr and the precipitate was collected by filtration. The obtained crystals were washed successively with water and ethanol and dried to give the title compound as crystals (20.2 g, 131 mmol).
¹H-NMR(CDCl₃) δ: 2.21(3H, s), 3.18(3H, m), 3.47(3H, s),
6.96(1H, s).
MS(ESI) m/z [MH]+ 155.2

### Reference Example 2

### 2-benzyl-4-(N,N-dimethylaminomethylene)-5-oxazolinone

To phenaceturic acid (10.0 g, 51.8 mmol) were added chloroform (30.0 ml), phosphorus oxychloride (20.0 g, 130 mmol) and N,N-dimethylformamide (10.0 g, 137 mmol) in an ice bath and the mixture was stirred at 45°C for 1.5 hrs. The reaction mixture was concentrated under reduced pressure, and added dropwise to 28% aqueous ammonia (65 ml) while maintaining not more than 10°C. The mixture was extracted with chloroform, and the organic layer was washed with water and saturated aqueous sodium chloride solution and concentrated to dryness. The concentrate was washed with isopropyl alcohol, and the precipitate was collected by filtration, is washed with isopropyl alcohol and dried to give the title compound as crystals (9.90 g, 43.3 mmol).
¹H-NMR(CDCl₃) δ: 3.17(3H, s), 3.48(3H, m), 3.83(2H, s),
6.97(1H, s), 7.23-7.36(5H, m)
MS(ESI) m/z [MH]+ 231.5

### Reference Example 3

### 2-methyl-4-(morpholinomethylene)-5-oxazolinone

To N-acetylglycine (0.50 g, 4.27 mmol) were added phosphorus oxychloride (995 µl, 10.7 mmol) and N-formylmorpholine (1.23 g, 10.7 mmol) in an ice bath, and the mixture was stirred at 45°C for 1.5 hrs. The reaction mixture was concentrated under reduced pressure, and added dropwise to 28% aqueous ammonia (5 ml) and water (5 ml) while maintaining not more than 10°C. The reaction mixture was stirred in an ice bath for 1 hr and the precipitate was collected by filtration. The obtained crystals were washed successively with water and ethanol, and dried to give the title compound as crystals (0.52 g, 2.65 mmol) .
¹H-NMR(CDCl₃) δ: 2.21(3H, s), 3.47(2H, br), 3.79(4H, t,
J=4.8Hz), 4.27(2H, s), 6.92(1H, s)
MS(ESI) m/z [MH]+ 196.9

### Reference Example 4

### 2-benzyl-4-(N,N-dimethylaminomethylene)-5-oxazolinone

To phenaceturic acid (1.00 g, 5.18 mmol) were added toluene (10.0 ml) and N,N'-dicyclohexylcarbodiimide (1.07 g, 5.19 mmol) and the mixture was stirred at room temperature overnight. The precipitate was collected by filtration and N,N-dimethylformamide dimethylacetal (0.68 g, 5.71 mmol) was added. The mixture was stirred overnight. The reaction mixture was washed with saturated aqueous sodium chloride solution and concentrated to dryness. Isopropyl alcohol was added to the concentrate to allow crystal precipitation, and the precipitate was collected by filtration, washed with isopropyl alcohol and vacuum dried to give the title compound as crystals (0.78 g, 3.39 mmol).

### Example 1

### (5-acetylamino-6-oxo-2-phenyl-1,6-dihydropyrimidin-1-yl)acetic acid

N-(carboxymethyl)phenylamidine (108 mg, 0.61 mmol) and 28% sodium methoxide methanol solution (117 mg, 0.61 mmol) were stirred in methanol (3 ml) and the mixture was concentrated to dryness. N,N-dimethylformamide (3 ml) and 4-(N,N-dimethylaminomethylene)-2-methyl-5-oxazolinone (94.0 mg, 0.61 mmol) were added and the mixture was stirred overnight at 130°C. The solvent was evaporated and ethyl acetate and water were added to allow partitioning. Ethyl acetate was added to the aqueous layer, and the mixture was adjusted to pH 2.0 with aqueous hydrochloric acid solution and extracted. The organic layer was concentrated to dryness and purified by silica gel column chromatography to give a crystal (54.0 mg, 0.19 mmol) of the title compound.
H-NMR (DMSO-d₆) δ: 2.15(3H, s), 4. 52 (2H, s), 7.48-7.56(5H, m),
8.83 (1H, s), 9.58(1H, s)
MS(ESI) m/z [MH]+ 288.2, [MH]- 286.1

### Example 2

### 5-acetylamino-6-oxo-2-phenyl-1-(succinimidoxycarbonylmethyl)-1,6-dihydropyrimidine

(5-Acetylamino-6-oxo-2-phenyl-1,6-dihydropyrimidin-1-yl)acetic acid (300 mg, 1.04 mmol), acetonitrile (5 ml) and N-methylmorpholine (121 mg, 1.20 mmol) were mixed and ethyl chloroformate (125 mg, 1.15 mmol) was added. The mixture was stirred under ice-cooling for 30 min. N-Hydroxysuccinimide (148 mg, 1.29 mmol) was added and the mixture was stirred for 5 hrs. The reaction mixture was concentrated under reduced pressure and ethyl acetate was added. The mixture was washed successively with saturated aqueous sodium hydrogen carbonate solution and saturated aqueous sodium chloride solution and concentrated to dryness. The residue was subjected to slurry washing with water (5 ml) and the precipitate was collected by filtration. The crystals were dried to give the title compound as color crystals (0.31 g, 0.81 mmol).
¹H-NMR(CDCl₃) δ: 2.23(3H, s), 2.88(4H, s), 4.94 (2H, s), 7.50-7.56(5H, m), 8.04(1H, s), 9.10(1H, s)
MS(ESI) m/z [MH]+ 385.2, [MH]- 383.2

### Example 3

### (S)-2-[2-(5-acetylamino-6-oxo-2-phenyl-1,6-dihydropyrimidin-1-yl)acetylamino]-3-plienylpropionic acid

L-Phenylalanine (100 mg, 0.61 mmol) was added to 50% aqueous acetonitrile (3 ml), and the mixture was adjusted to pH 8 with 6N aqueous sodium hydroxide solution to allow dissolution. To this solution was added dropwise a solution of 5-acetylamino-6-oxo-2-phenyl-1-(succinimidoxycarbonylmethyl)-1,6-dihydropyrimidine (176 mg, 0.46 mmol) in acetonitrile (1 ml) under ice-cooling. The mixture was stirred at room temperature for 3 hrs. The reaction mixture was concentrated and adjusted to pH 2 with aqueous hydrogen chloride solution to allow crystal precipitation and stirred for 2 hrs. The precipitate was collected by filtration, washed and vacuum dried to give the title compound as white crystals (190 mg, 0.44 mmol).
¹H-NMR(DMSO-d₆) δ: 2.14(3H, s), 2.84-2.87(1H, dd, J1=13.8Hz, J2=5.0Hz), 3.01(1H, dd, J1=13.8Hz, J2=8.7Hz), 4.41-4.48(3H, m), 7.12-7.14(2H, d, J=7.8Hz), 7.21-7.25(3H, m), 7.43-7.52(5H, m), 8.53(1H, d, J=7.8Hz), 8.80(1H, s), 9.52(1H, s)
MS(ESI) m/z [MH]+ 435.2, [MH]- 433.3

### Example 4

### (S)-2-[(6-oxo-2-phenyl-5-phenylacetylamino-1,6-dihydropyrimidin-1-yl)acetylamino]-3-phenylpropionic acid

In the same manner as in Example 3 except that 6-oxo-2-phenyl-5-phenylacetylamino-1-(succinimideoxycarbonylmethyl)-1,6-dihydropyrimidine was used instead of 5-acetylamino-6-oxo-2-phenyl-1-(succinimidoxycarbonylmethyl)-1,6-dihydropyrimidine, the title compound was obtained.
¹H-NMR(DMSO-d₆) δ :2.85(1H, dd, J1=14.1Hz, J2=5.1Hz), 3.02(1H, dd, J1=14.1Hz, J2=5.1Hz), 3.83(2H, s), 4.45(3H, m), 7.15-7.52 (15H, m), 8.53(1H, d, J=7.8Hz), 8.80(1H, s), 9.64(1H, s)
MS(ESI) m/z [MH] + 511.3, [MH]- 509.4

### Example 5

### Methyl (S)-2-[2-(5-acetylamino-6-oxo-2-phenyl-1,6-dihydropyrimidin-1-yl)acetylamino]-3-methylbutyrate

To (5-acetylamino-6-oxo-2-phenyl-1,6-dihydropyrimidin-1-yl)acetic acid (250 mg, 0.87 mmol) were added tetrahydrofuran (5 ml) and N-methylmorpholine (0.10 ml, 0.91 mmol) to allow dissolution. Under ice-cooling, ethyl chloroformate (85.3 µl, 0.91 mmol) was added and the mixture was stirred for 20 min. L-valine methyl ester hydrochloride (146 mg, 0.87 mmol) and N-methylmorpholine (0.10 ml, 0.91 mmol) were added and the mixture was stirred for 2 hrs. Ethyl acetate was added to the reaction mixture and the mixture was washed successively with 1N aqueous hydrochloric acid, saturated aqueous sodium hydrogen carbonate solution and saturated aqueous sodium chloride solution. The organic layer was concentrated to dryness to give the title compound (0.18 g, 0.45 mmol).
¹H-NMR(CDCl₃) δ : 0.92(3H, d, J=6. 9Hz), 0.94(3H, d, J=6.9Hz), 2.16-2.19(1H, m), 2.21(3H, S), 3.75(3H, s), 4.58(3H, m), 6.37(1H, d, J=8.6Hz), 7.43-7.50(3H, m), 7.55-7.57(2H, m), 8.01(1H, s) 9.10(1H, s)

### Example 6

### N-[2-(5-tert-butyl-1,3,4-oxadiazol-2-yl)-1-methyl-2-oxoethyl]-(6-oxo-2-phenyl-5-phenylacetylamino-1,6-dihydropyrimidin-1-yl)acetamide

To (6-oxo-2-phenyl-5-phenylacetylamino-1,6-dihydropyrimidin-1-yl)acetic acid (600 mg, 1.65 mmol) were added tetrahydrofuran (8 ml) and N-methylmorpholine (0.20 ml, 1.82 mmol) to allow dissolution. Under ice-cooling, ethyl chloroformate (0.165 ml, 1.73 mmol) was added and the mixture was stirred for 20 min. 2-Amino-1-(5-tert-butyl-1,3,4-oxadiazol-2-yl)-1-propanone hydrochloride (386 mg, 1.65 mmol) and N-methylmorpholine (0.20 ml, 1.82 mmol) were added and the mixture was stirred for 1 hr. Methyl-tert-butyl ether was added to the reaction mixture and the mixture was washed successively with 1N aqueous hydrochloric acid, saturated aqueous sodium hydrogen carbonate solution and saturated aqueous sodium chloride solution. The organic layer was concentrated to dryness to give the title compound (0.61 g, 1.12 mmol).
¹H-NMR(CDCl₃) δ :1.48(9H, s), 1.53(3H, d, J=4.2Hz), 3.76(2H,s), 4.57(2H, s), 5.43-5.51(1H, m), 6.76(1H, d, J=6.8Hz), 7.25-7.32(5H, m), 7.43-7.53(5H, m), 8.05(1H, s), 9.09(1H, s)
MS(ESI) m/z [MH]+ 543.4, [MH]- 541.4

### Example 7

### N-[2-(5-tert-butyl-1,3,4-oxadiazol-2-yl)-1-methyl-2-oxoethyl]-(5-acetylamino-6-oxo-2-phenyl-1,6-dihydropyrimidin-1-yl)acetamide

In the same manner as in Example 6 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, the title compound was obtained.
¹H-NMR(CDCl₃) δ :1.48(9H, s), 1.59 (3H, d, J=7.3Hz), 2.23(3H, s), 4.62(2H, s), 5.48-5.54(1H, m), 6.79(1H, d, J=6.9Hz), 7.47-7.56(5H, m), 8.02(1H, s), 9.08(1H, s)
MS(ESI) m/z [MH]+ 467.2, [MH]- 465.4

### Example 8

### N-[2-methyl-1-(2-thiazolylcarbonyl)propyl]-(6-oxo-2-phenyl-5-phenylacetylamino-1,6-dihydropyrimidin-1-yl)acetamide

To (6-oxo-2-phenyl-5-phenylacetylamino-1,6-dihydropyrimidin-1-yl)acetic acid (400 mg, 1.10 mmol) were added tetrahydrofuran (6 ml) and N-methylmorpholine (0.133 ml, 1.21 mmol) for dissolution. Under ice-cooling, ethyl chloroformate (0.11 ml; 1.15 mmol) was added and the mixture was stirred for 20 min. 2-Amino-3-methyl-1-(2-thiazolyl)-1-butanone hydrochloride (250 mg, 1.13 mmol) and N-methylmorpholine (0.133 ml, 1.21 mmol) were added and the mixture was stirred for 1 hr. Ethyl-tert-butyl ether was added to the reaction mixture, and the mixture was washed successively with 1N aqueous hydrochloric acid, saturated aqueous sodium hydrogen carbonate solution and saturated aqueous sodium chloride solution. The organic layer was concentrated to dryness to give the title compound (556 mg, 1.05 mmol).
¹H-NMR(CDCl₃) δ : 0.76(3H, d, J=6.8Hz), 0.99(3H, d, J=6.8Hz), 2.37-2.45(1H, m), 3.75(2H, s), 4.11(1H, dd, J1=14.3Hz, J2=7.1Hz), 4.60-4.70(2H, m), 5.65-5.68(1H, dd, J1=8.9Hz, J2=4.9Hz), 7.05(1H, d, J=8.9Hz), 7.23-7.53(10H, m), 7.69(1H, d, J=3.0Hz), 7.99(1H, d, J=3.0Hz), 8.16(1H, s), 9.08(1H, s)
MS(ESI) m/z [MH]+ 530.3, [MH]- 528.3

### Example 9

### N-[2-methyl-1-(2-thiazolylcarbonyl)propyl]-(5-acetylamino-6-oxo-2-phenyl-1,6-dihydropyrimidin-1-yl)acetamide

In the same manner as in Example 8 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, the title compound was obtained.
¹H-NMR(CDCl₃) δ : 0.84(3H, d, J=6.9Hz), 1.05 (3H, d, J=6.9Hz), 2.22(3H, s), 2.48(1H, m), 4.61(1H, d, J=16Hz), 4.68(1H, d, J=16Hz), 5,67(1H, dd, J1=8.8Hz, J2=4.8Hz), 6.78(1H, d, J=8.8Hz), 7.41-7.46(3H, m), 7.54-7.56(2H, m), 7.73(1H, d, J=3.0Hz), 8.00(1H, s), 8.04(1H, d, J=3.0Hz), 9.09(1H, s)
MS(ESI) m/z [MH]+ 454.2, [MH]- 452.4

### Example 10

### (S)-N-[1-benzyl-3-chloro-2-oxopropyl]-(5-acetylamino-6-oxo-2-phenyl-1,6-dihydropyrimidin-1-yl)acetamide

In the same manner as in Example 6 except that (5-acetylamino-6-oxo-2-phenyl-1,6-dihydropyrimidin-1-yl)acetic acid was used instead of (6-oxo-2-phenyl-5-phenylacetylamino-1,6-dihydropyrimidin-1-yl)acetic acid, and (S)-3-amino-1-chloro-4-phenyl-2-butanone hydrochloride was used instead of 2-amino-1-(5-tert-butyl-1,3,4-oxadiazol-2-yl)-1-propanone hydrochloride, the title compound was obtained.
¹H-NMR(CDC1₃) δ :2.24(3H, s), 3.02-3.07(1H, dd, J1=14.0Hz, J2=6.9Hz), 3.10-3.16(1H, dd, J1=14.0Hz, J2=6.9Hz), 4.00(1H, d, J=16.1Hz), 4.15(1H, d, J=16.1Hz) 4.48(1H, d, J=15.6Hz), 4.56(1H, d, J=15.6Hz), 4.98-5.03(1H, m), 6.51(1H, d, J=7.2Hz), 7.10-7.12(2H, m), 7.26-7.29(3H, m), 7.45-7.49(5H, m), 7.96(1H, s), 9.10(1H, s)
MS(ESI) m/z [MH]+ 467.2, [MH]- 465.2

### Example 11

### (S)-2-[(5-amino-6-oxo-2-phenyl-1,6-dihydropyrimidin-1-yl)acetylamino]-3-phenylpropionic acid

To (S)-2-[(6-oxo-2-phenyl-5-phenylacetylamino-1,6-dihydropyrimidin-1-yl)acetylamino]-3-phenylpropionic acid (135 mg, 0.264 mmol) were added water (10 ml) and potassium dihydrogen phosphate (155 mg, 1.14 mmol) and the mixture was adjusted to pH 7.6 with aqueous sodium hydroxide solution. Penicillin amidase beads (23.0 mg, Sigma) were added and the mixture was stirred overnight at 37°C. Acetonitrile was added to the obtained reaction mixture and the precipitate was filtered off. The filtrate was concentrated and aqueous hydrochloric acid solution was added to adjust to pH 2.0 to allow crystal precipitation. The precipitate was collected by filtration, washed and vacuum dried to give the title compound as crystals (85.0 mg, 0.217 mmol).
¹H-NMR(DMSO-d₆) δ :2.83-2.88(1H, m), 3.00-3.03(1H, m), 4.33-4.47(3H, m), 5.14(2H, br), 7.14-7.45(10H, m), 8.44(1H, d, J=7.8Hz)
MS(ESI) m/z [MH]+ 393.3, [MH]- 391.2

### Example 12

### N-[2-methyl-1-(2-thiazolylcarbonyl)propyl]-(5-amino-6-oxo-2-phenyl-1,6-dihydropyrimidin-1-yl)acetamide

N-[2-Methyl-1-(2-thiazolylcarbonyl)propyl]-(6-oxo-2-phenyl-5-phenylacetylamino-1,6-dihydropyrimidin-1-yl)acetamide (150 mg, 0.283 mmol) was suspended in 0.05 M potassium phosphate buffer (pH 7.4, 15.0 ml) and Triton X-100 (trademark) (2.00 µl) and aqueous penicillin amidase solution (Sigma) were added. The mixture was stirred at 37°C for 5 days. The precipitate of the obtained reaction mixture was collected by filtration, washed and vacuum dried to give the title compound as crystals (114 mg, 0.277 mmol).
¹H-NMR(CDCl₃) δ :0.84(3H, d, J=6.8Hz), 1.05(3H, d, J=6.82Hz), 2.45-2.50(1H, m), 4.04(2H, s), 4.58(1H, d, J=15.5Hz), 4.66(1H, d, J=15.5Hz), 5.64(1H, dd, J1=8.9Hz, J2=4.8Hz), 6.88(1H, d, J=8.9Hz), 7.40-7.52(6H,m), 7.72(1H, d, J=3.1Hz), 8.03(1H, d, J=3.1Hz)
MS(ESI) m/z [MH]+ 412.2, [MH]- 410.0

### Example 13

### N-[2-(5-tert-butyl-1,3,4-oxadiazol-2-yl)-1-methyl-2-oxoethyl]-(5-amino-6-oxo-2-phenyl-1,6-dihydropyrimidin-1-yl)acetamide

N-[2-(5-tert-Butyl-1,3,4-oxadiazol-2-yl)-1-methyl-2-oxoethyl]-(6-oxo-2-phenyl-5-phenylacetylamino-1,6-dihydropyrimidin-1-yl)acetamide (150 mg, 0.277 mmol) was suspended in 0.05 M potassium phosphate buffer (pH 7.4, 15.0 ml) and Triton X-100 (trademark) (2.00 µl) and aqueous penicillin amidase solution (Sigma) were added. The mixture was stirred overnight at 37°C. The precipitate pf the obtained reaction mixture was collected by filtration, washed and vacuum dried to give the title compound as crystals (114 mg, 0.269 mmol).
¹H-NMR(CDCl₃) δ : 1.48(9H, s), 1.57(3H, d, J=7.3Hz), 4.48(1H, d, J=15.5Hz), 4.64(1H, d, J=15. 5Hz), 4.47(1H, m), 7.01(1H, d, J=6.6Hz), 7.43-7.53(6H, m)
MS(ESI) m/z [MH]+ 425.3, [MH]- 423.1

### Example 14

### Methyl (S)-2-[(5-amino-6-oxo-2-phenyl-1,6-dihydropyrimidin-1-yl)acetylamino]-3-methylbutyrate hydrochloride

Methyl (S)-2-[(5-acetylamino-6-oxo-2-phenyl-1,6-dihydropyrimidin-1-yl)acetylamino]-3-methylbutyrate (100 mg, 0.25 mmol) was dissolved in methanol (5 ml) and a 10% solution (0.5 ml) of hydrogen chloride in methanol was added. The mixture was stirred overnight at room temperature. The reaction mixture was concentrated to dryness and methyl-tert-butyl ether was added. The precipitate was filtered, washed with methyl-tert-butyl ether and dried to give the title compound as crystals (90.0 mg, 0.228 mmol).
¹H-NMR (DMSO-d₆) δ : 0.80(3H, d, J=3.0Hz), 0.82(3H, d, J=3.0Hz), 1.97-2.02(1H, m), 3.64(3H, s), 4.20(1H, dd, J1=8.4Hz, J2=6.2Hz), 4.53(2H, s), 7.37(1H, s), 7.48-7.55 (5H, m), 8.51(1H, d, J=8.4Hz)
MS(ESI) m/z [MH]+ 359.4, [MH]- 393.1

### Example 15

### N-[2-methyl-1-(2-thiazolylcarbonyl)propyl]-(5-amino-6-oxo-2-phenyl-1,6-dihydropyrimidin-1-yl)acetamide hydrochloride

N-[2-Methyl-1-(2-thiazolylcarbonyl)propyl]-(5-acetylamino-6-oxo-2-phenyl-1,6-dihydropyrimidin-1-yl)acetamide (100 mg, 0.220 mmol) was dissolved in methanol (5 ml) and a 10% solution (0.5 ml) of hydrogen chloride in methanol was added. The mixture was stirred overnight at 60°C. The reaction mixture was concentrated to dryness and methyl-tert-butyl ether was added. The precipitate was filtered, washed with methyl-tert-butyl ether and dried to give the title compound as crystals (93.0 mg, 0.208 mmol).
¹H-NMR(DMSO-d₆) δ :0.80 (3H, d, J=6.9Hz), 0.87 (3H, d, J=6.9Hz), 2.28-2.33(1H, m), 4.62(2H, s), 5.45(1H, dd, J1=8.1Hz, J2=5.5Hz), 7.51- 7.62(6H,m), 8.20(1H, d, J=3.0Hz), 8.30(1H, d, J=3.0Hz), 8.75(1H, d, J=8.1Hz)
MS(ESI) m/z [MH]+ 412.3, [MH]- 446.2

### Example 16

### (S)-N-[1-benzyl-3-chloro-2-oxopropyl]-(5-amino-6-oxo-2-phenyl-1,6-dihydropyrimidin-1-yl)acetamide hydrochloride

In the same manner as in Example 14 except that (S)-N-[1-benzyl-3-chloro-2-oxopropyl]-(5-acetylamino-6-oxo-2-phenyl-1,6-dihydropyrimidin-1-yl)acetamide was used instead of methyl (S)-2-[(5-acetylamino-6-oxo-2-phenyl-1,6-dihydropyrimidin-1-yl)acetylamino]-3-methylbutyrate, the title compound was obtained.
¹H-NMR(DMSO-d₆) δ :2.78-2.84(1H, m), 3.07-3.11(1H, m), 4.43(2H, s), 4.55-4.60(3H, m), 7.13-7.23(5H, m), 7.43-7.58(5H, m), 7.59-7.60(1H, m), 8.96(1H, d, J=7.4Hz)
MS(ESI) m/z [MH]+ 425.2, [MH]- 459.2

### Industrial Applicability

The present invention provides an efficient process for preparing compound (III), and a process for advantageously preparing compound (XII) or an N-protected form thereof, which is a final object compound useful as an enzyme inhibitor, by using compound (III) as a starting material compound or an intermediate compound.

In compound (VIII) prepared by the process of the present invention, the amino group at the 5-position of pyrimidine ring is protected by a protecting group that can be eliminated under mild conditions. Therefore, deprotection can be easily carried out without inducing decomposition of the resultant product and the like in the final stage to reach compound (XII).

Therefore, the preparation process of the present invention does not require elimination of a protecting group prior to an amidation reaction, unlike conventional processes using an intermediate compound wherein the 5-position of pyrimidine ring is protected by a benzoyl group, and as a result, suppresses side reactions such as dimerization and the like caused by an amidation reaction which is carried out while an amino group at the 5-position of the pyrimidine ring is free, and the object compound having high quality can be obtained in a high yield.

This application is based on a patent application No. 2003-374043 filed in Japan, the contents of which are incorporated in full herein by this reference.

## Claims

1. A process for preparing a compound represented by the formula (III) : 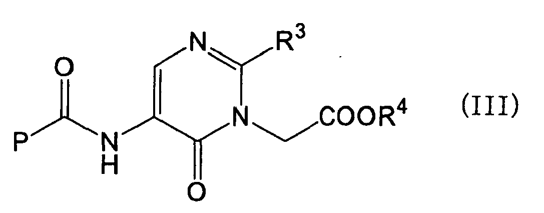 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), and R⁴ is a hydrogen atom, an alkyl group or an aralkyl group, or a salt thereof, which comprises reacting a compound represented by the formula (I): 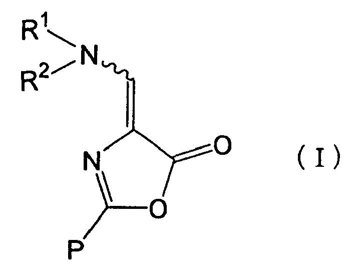 wherein R¹ and R² are the same or different and each independently is an alkyl group, or optionally form an aliphatic heterocycle together with the adjacent nitrogen atom, a wavy line shows a cis form or a trans form or a mixture thereof, and P is as defined above, with a compound represented by the formula (II): 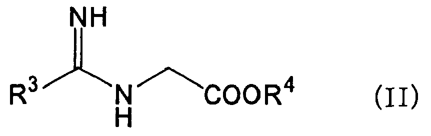 wherein each symbol is as defined above, or a salt thereof.

2. A process for preparing a compound represented by the formula (IV): 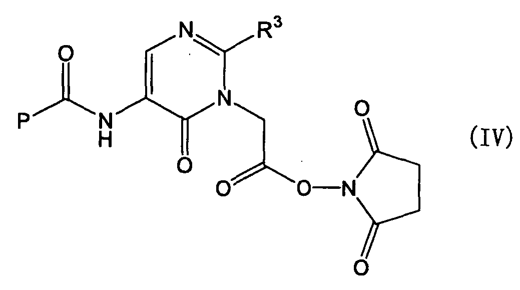 wherein P is a hydrogen atom, an alkyl group, an alkenyl group, an aralkyl group optionally having substituent(s) or a haloalkyl group, and R³ is an alkyl group optionally having substituent(s) or a phenyl group optionally having substituent(s), which comprises reacting a compound represented by the formula (I): 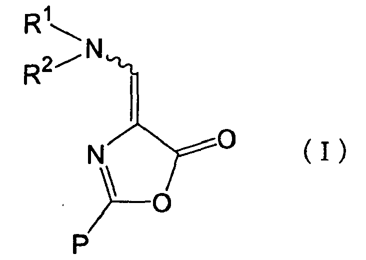 wherein R¹ and R² are the same or different and each independently is an alkyl group, or optionally form an aliphatic heterocycle together with the adjacent nitrogen atom, a wavy line shows a cis form or a trans form or a mixture thereof, and P is as defined above, with a compound represented by the formula (II): 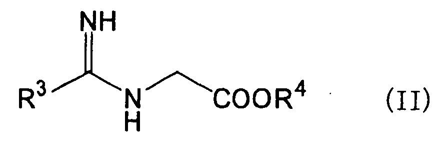 wherein R⁴ is a hydrogen atom, an alkyl group or an aralkyl group, and R³ is as defined above, or a salt thereof to give a compound represented by the formula (III): 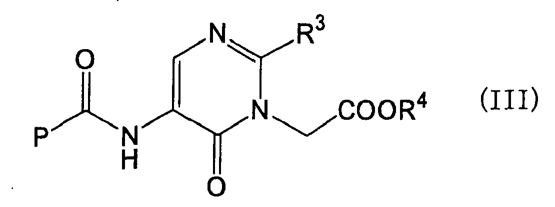 wherein each symbol is as defined above, or a salt thereof; and condensing the obtained compound represented by the formula (III) or a salt thereof with N-hydroxysuccinimide after converting R⁴ to a hydrogen atom when it is other than a hydrogen atom.

3. The process of claim 1 or 2, wherein P is a methyl group, an ethyl group or a benzyl group.

4. The process of claim 1 or 2, wherein R³ is a phenyl group optionally having substituent(s) or a methyl group.

5. The process of claim 1 or 2, wherein R⁴ is a hydrogen atom, a methyl group or a tert-butyl group.

6. A process for preparing a compound represented by the formula (VI): 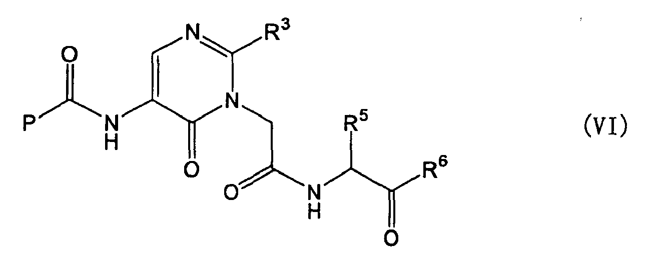 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and R⁶ is a hydrogen atom, a hydroxyl group, an alkoxy group, an aralkyloxy group or -N(R⁷)-OR⁸ (wherein R⁷ and R⁸ are the same or different and each independently is an alkyl group), or a salt thereof, which comprises the following Steps (a), (b) and (c):
Step (a): reacting a compound represented by the formula (I): 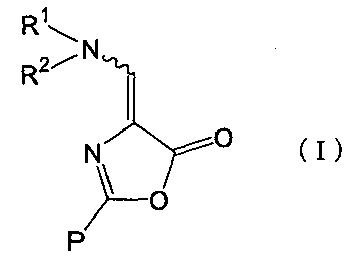 wherein P is as defined above, R¹ and R² are the same or different and each independently is an alkyl group, or optionally form an aliphatic heterocycle together with the adjacent nitrogen atom, and a wavy line shows a cis form or a trans form or a mixture thereof, with a compound represented by the formula (II): 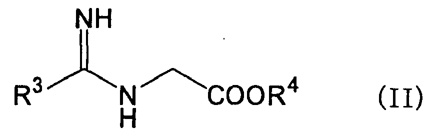 wherein R³ is as defined above, and R⁴ is a hydrogen atom, an alkyl group or an aralkyl group, or a salt thereof to give a compound represented by the formula (III): 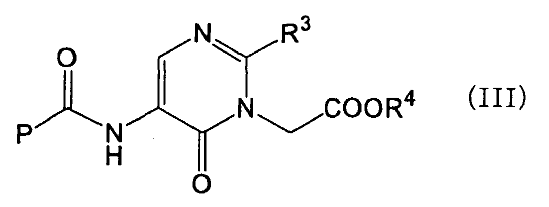 wherein each symbol is as defined above, or a salt thereof; Step (b): condensing the obtained compound represented by the formula (III) or a salt thereof with N-hydroxysuccinimide after converting R⁴ to a hydrogen atom when it is other than a hydrogen atom, to give a compound represented by the formula (IV) : 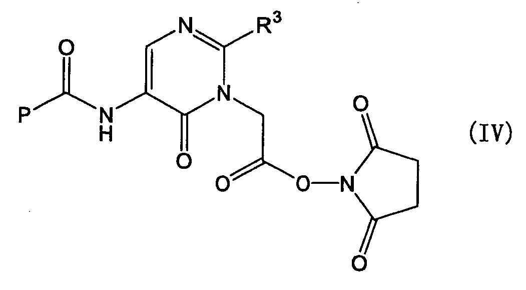 wherein each symbol is as defined above; and
Step (c): reacting the obtained compound represented by the formula (IV) with a compound represented by the formula (V): 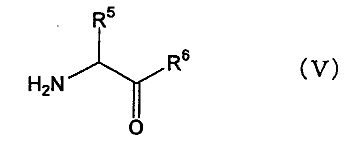 wherein each symbol is as defined above, or a salt thereof to give a compound represented by the formula (VI) or a salt thereof.

7. A process for preparing a compound represented by the formula (VI): 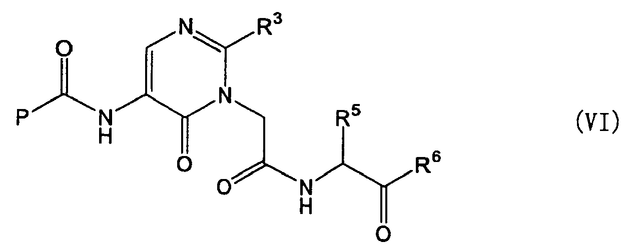 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and R⁶ is a hydrogen atom, a hydroxyl group, an alkoxy group, an aralkyloxy group or -N(R⁷)-OR⁸ (wherein R⁷ and R⁸ are the same or different and each independently is an alkyl group), or a salt thereof, which comprises reacting a compound represented by the formula (I): 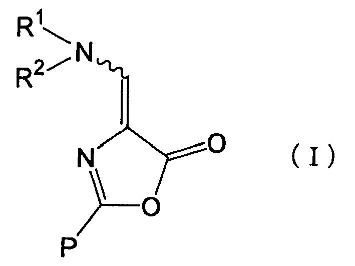 wherein P is as defined above, R¹ and R² are the same or different and each independently is an alkyl group, or optionally form an aliphatic heterocycle together with the adjacent nitrogen atom, and a wavy line shows a cis form or a trans form or a mixture thereof, with a compound represented by the formula (II): 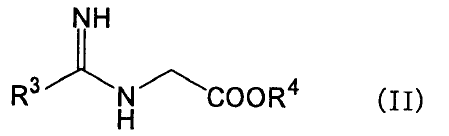 wherein R³ is as defined above, and R⁴ is a hydrogen atom, an alkyl group or an aralkyl group, or a salt thereof to give a compound represented by the formula (III): 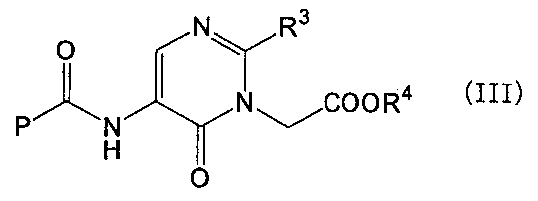 wherein each symbol is as defined above, or a salt thereof, and condensing the obtained compound represented by the formula (III) or a salt thereof with a compound represented by the formula (V): 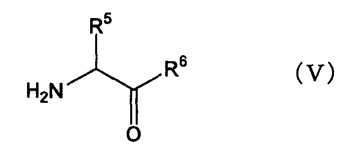 wherein each symbol is as defined above, or a salt thereof after converting R⁴ to a hydrogen atom when it is other than a hydrogen atom.

8. The process of claim 6 or 7, wherein P is a methyl group, an ethyl group or a benzyl group.

9. The process of claim 6 or 7, wherein R³ is a phenyl group optionally having substituent(s) or a methyl group.

10. The process of claim 6 or 7, wherein R⁴ is a hydrogen atom, a methyl group or a tert-butyl group.

11. A process for preparing a compound represented by the formula (VII) : 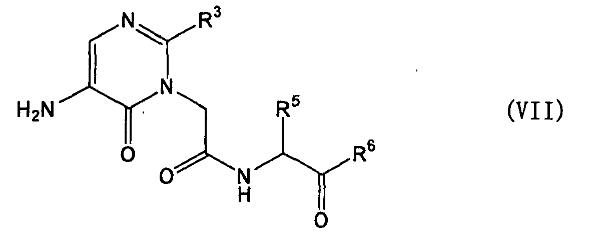 wherein R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and R⁶ is a hydrogen atom, a hydroxyl group, an alkoxy group, an aralkyloxy group or -N(R⁷)-OR⁸ (wherein R⁷ and R⁸ are the same or different and each independently is an alkyl group), or a salt thereof, which comprises deprotecting a compound represented by the formula (VI) : 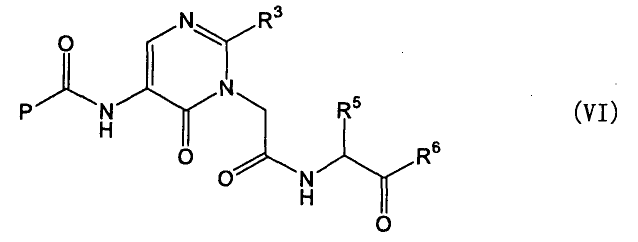 wherein P is a hydrogen atom, an alkyl group, an alkenyl group, an aralkyl group optionally having substituent(s) or a haloalkyl group, and other symbols are as defined above, or a salt thereof, which is obtained by the process of any one of claims 6 to 10.

12. The process of claim 11, wherein the deprotection is carried out by an enzyme reaction.

13. The process of claim 11, wherein the deprotection is carried out under acidic conditions.

14. A process for preparing a compound represented by the formula (XII): 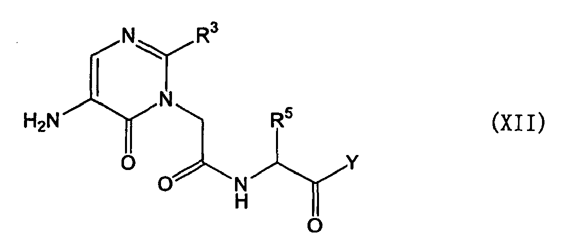 wherein R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and Y is a heterocyclic group optionally having substituent(s), an acyl group optionally having substituent(s) or a haloalkyl group, or a salt thereof, which comprises the following Steps (h), (i) and (j) :
Step (h): protecting an amino group of a compound represented by the formula (VII): 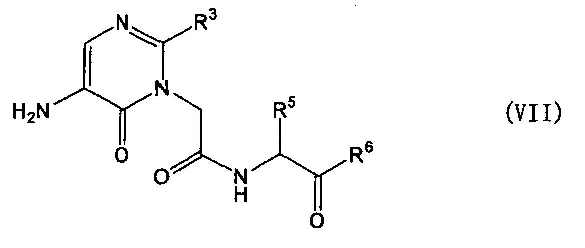 wherein R⁶ is a hydrogen atom, a hydroxyl group, an alkoxy group, an aralkyloxy group or -N(R⁷)-OR⁸ (wherein R⁷ and R³ are the same or different and each independently is an alkyl group), and other symbols are as defined above, or a salt thereof, which is obtained by the process of any one of claims 11 to 13 to give a compound represented by the formula (X): 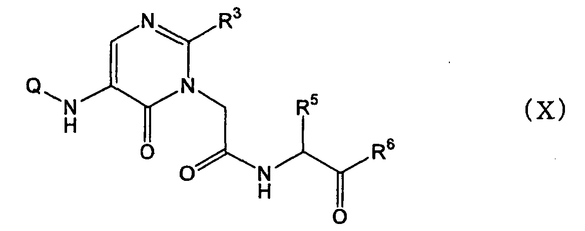 wherein Q is an amino-protecting group other than an acyl group, and other symbols are as defined above, or a salt thereof;
Step (i): converting a group represented by R⁶ of the obtained compound represented by the formula (X) or a salt thereof to a group represented by Y to give a compound represented by the formula (XI): 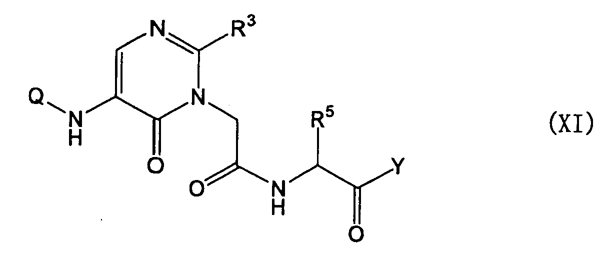 wherein each symbol is as defined above, or a salt thereof; and Step (j): deprotecting the obtained compound represented by the formula (XI) or a salt thereof to give a compound represented by the formula (XII) or a salt thereof.

15. A process for preparing a compound represented by the formula (VIII): 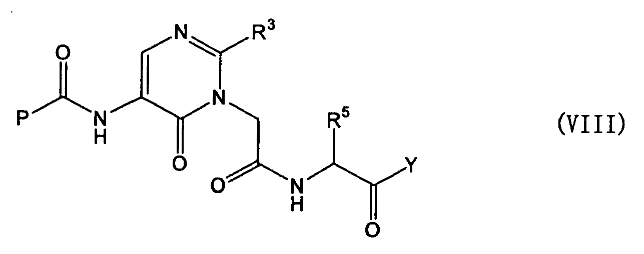 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and Y is a heterocyclic group optionally having substituent(s), an acyl group optionally having substituent(s) or a haloalkyl group, or a salt thereof, which comprises the following Steps (a), (b), (c) and (e):
Step (a) : reacting a compound represented by the formula (I): 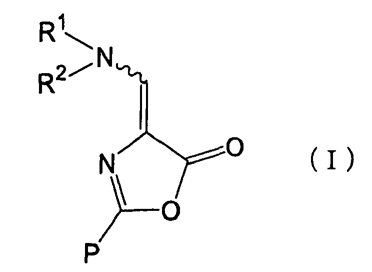
wherein P is as defined above, R¹ and R² are the same or different and each independently is an alkyl group, or optionally form an aliphatic heterocycle together with the adjacent nitrogen atom, and a wavy line shows a cis form or a trans form or a mixture thereof, with a compound represented by the formula (II) : 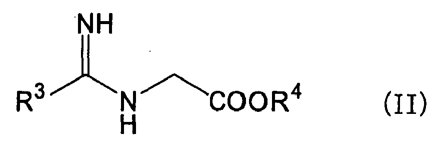 wherein R³ is as defined above, and R⁴ is a hydrogen atom, an alkyl group or an aralkyl group, or a salt thereof to give a compound represented by the formula (III): 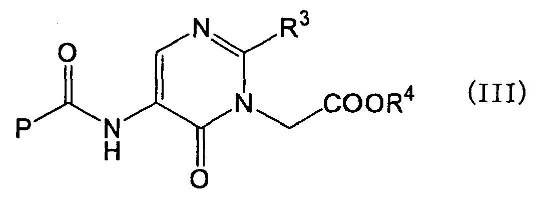 wherein each symbol is as defined above, or a salt thereof; Step (b): condensing the obtained compound represented by the formula (III) or a salt thereof with N-hydroxysuccinimide after converting R⁴ to a hydrogen atom when it is other than a hydrogen atom to give a compound represented by the formula (IV) : 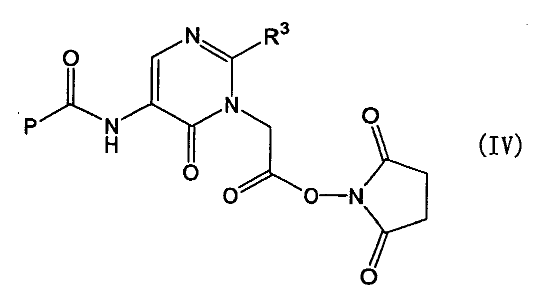 wherein each symbol is as defined above;
Step (c): reacting the obtained compound represented by the formula (IV) with a compound represented by the formula (V): 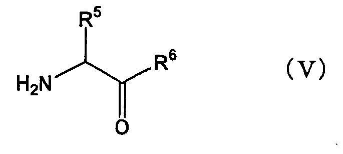 wherein R⁵ is as defined above, and R⁶ is a hydrogen atom, a hydroxyl group, an alkoxy group, an aralkyloxy group or -N(R⁷)-OR⁸ (wherein R⁷ and R⁸ are the same or different and each independently is an alkyl group), or a salt thereof to give a compound represented by the formula (VI): 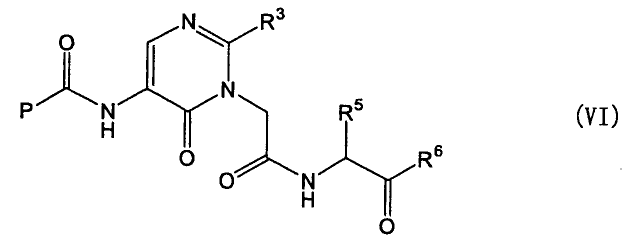 wherein each symbol is as defined above, or a salt thereof; and Step (e): converting a group represented by R⁶ of the obtained compound represented by the formula (VI) or a salt thereof to a group represented by Y to give a compound represented by the formula (VIII) or a salt thereof.

16. A process for preparing a compound represented by the formula (VIII) : 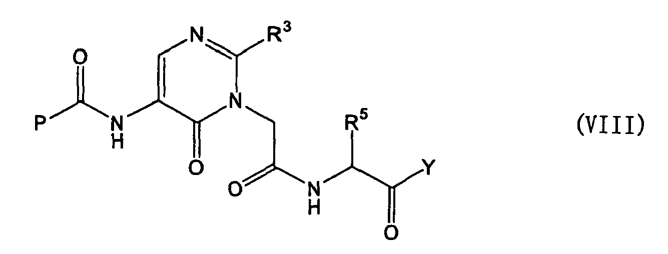 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and Y is a heterocyclic group optionally having substituent(s), an acyl group optionally having substituent(s) or a haloalkyl group, or a salt thereof, which comprises the following Steps (a), (d) and (e):
Step (a): reacting a compound represented by the formula (I): 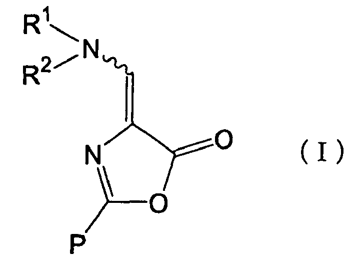 wherein P is as defined above, R¹ and R² are the same or different and each independently is an alkyl group, or optionally form an aliphatic heterocycle together with the adjacent nitrogen atom, and a wavy line shows a cis form or a trans form or a mixture thereof, with a compound represented by the formula (II) : 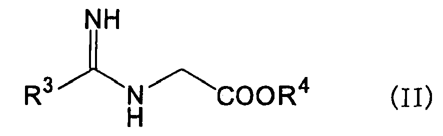 wherein R³ is as defined above, and R⁴ is a hydrogen atom, an alkyl group or an aralkyl group, or a salt thereof to give a compound represented by the formula (III): 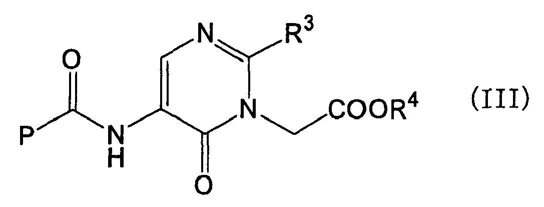 wherein each symbol is as defined above, or a salt thereof;
Step (d): condensing the obtained compound represented by the formula (III) or a salt thereof with a compound represented by the formula (V): 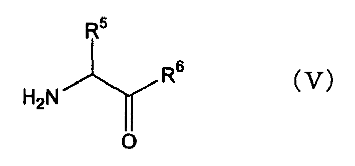 wherein R⁵ is as defined above, and R⁶ is a hydrogen atom, a hydroxyl group, an alkoxy group, an aralkyloxy group or -N(R⁷)-OR⁸ (wherein R⁷ and R⁸ are the same or different and each independently is an alkyl group), or a salt thereof after converting R⁴ to a hydrogen atom when it is other than a hydrogen atom, to give a compound represented by the formula (VI) : 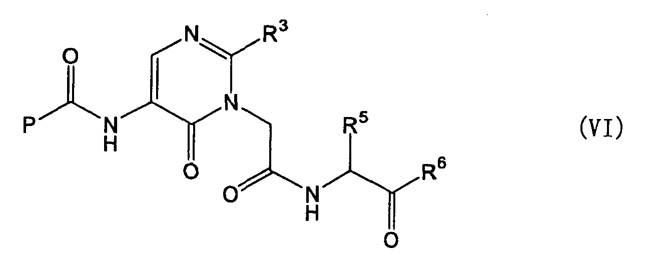 wherein each symbol is as defined above, or a salt thereof; and Step (e): converting a group represented by R⁶ of the obtained compound represented by the formula (VI) or a salt thereof to a group represented by Y to give a compound represented by the formula (VIII) or a salt thereof.

17. A process for preparing a compound represented by the formula (VIII): 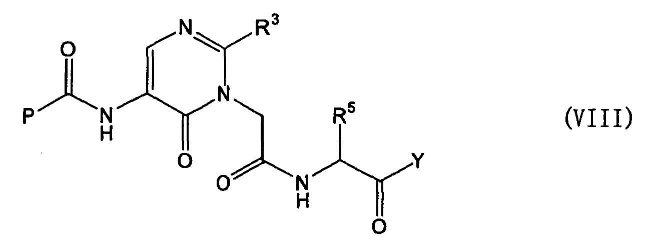 wherein P is a hydrogen atom, an alkyl group, an alkenyl group, an aralkyl group optionally having substituent(s) or a haloalkyl group, R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and Y is a heterocyclic group optionally having substituent(s), an acyl group optionally having substituent(s) or a haloalkyl group, or a salt thereof, which comprises reacting a compound represented by the formula (I): 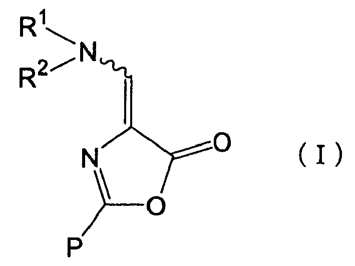 wherein P is as defined above, R¹ and R² are the same or different and each independently is an alkyl group, or optionally form an aliphatic heterocycle together with the adjacent nitrogen atom, and a wavy line shows a cis form or a trans form or a mixture thereof, with a compound represented by the formula (II): 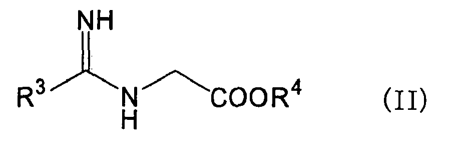 wherein R³ is as defined above, and R⁴ is a hydrogen atom, an alkyl group or an aralkyl group, or a salt thereof to give a compound represented by the formula (III): 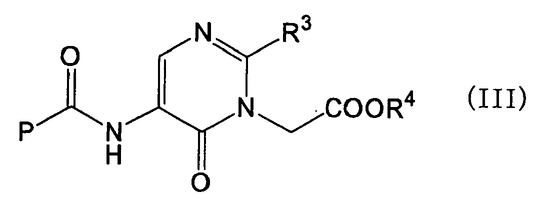 wherein each symbol is as defined above, or a salt thereof, and condensing the obtained compound represented by the formula (III) or a salt thereof with a compound represented by the formula (IX): 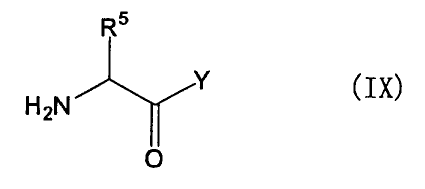 wherein each symbol is as defined above, or a salt thereof, after converting R⁴ to a hydrogen atom when it is other than a hydrogen atom.

18. The process of any one of claims 15 to 17, wherein P is a methyl group, an ethyl group or a benzyl group.

19. The process of any one of claims 15 to 17, wherein R³ is a phenyl group optionally having substituent(s) or a methyl group.

20. The process of any one of claims 15 to 17, wherein R⁴ is a hydrogen atom, a methyl group or a tert-butyl group.

21. A process for preparing a compound represented by the formula (XII): 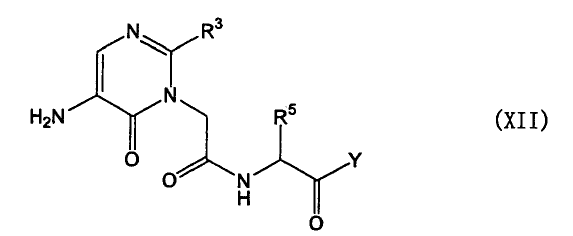 wherein R³ is an alkyl group optionally having substituent(s) or a phenyl group optionally having substituent(s), R⁵ is a hydrogen atom, an alkyl group optionally having substituent(s), an aralkyl group optionally having substituent(s) or an aryl group optionally having substituent(s), and Y is a heterocyclic group optionally having substituent(s), an acyl group optionally having substituent(s) or a haloalkyl group, or a salt thereof, which comprises deprotecting a compound represented by the formula (VIII): 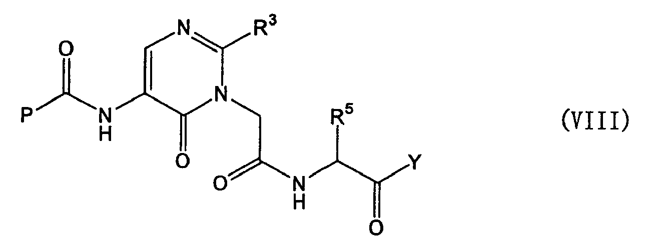 wherein P is a hydrogen atom, an alkyl group, an alkenyl group, an aralkyl group optionally having substituent(s) or a haloalkyl group, and other symbols are as defined above, or a salt thereof, which is obtained by the process of any one of claims 15 to 20.

22. The process of claim 21, wherein the deprotection is carried out by an enzyme reaction.

23. The process of claim 21, wherein the deprotection is carried out under acidic conditions.
